(19) Europäisches Patentamt
European Patent Office
Office européen des brevets

(11) Veröffentlichungsnummer: **0 591 780 A1**

(12) **EUROPÄISCHE PATENTANMELDUNG**

(21) Anmeldenummer: **93115419.9**

(51) Int. Cl.5: **C07D 417/04**, A01N 47/38

(22) Anmeldetag: **24.09.93**

(30) Priorität: **07.10.92 DE 4233713**

(43) Veröffentlichungstag der Anmeldung: **13.04.94 Patentblatt 94/15**

(84) Benannte Vertragsstaaten: **BE CH DE ES FR GB IT LI NL**

(71) Anmelder: **BAYER AG**

**D-51368 Leverkusen(DE)**

(72) Erfinder: **Fuchs, Rainer, Dr.**
**Am Rohm 107**
**D-42113 Wuppertal(DE)**
Erfinder: **Kanellakopulos, Johannes, Dr.**
**Mozartstrasse 94c**
**D-40724 Hilden(DE)**
Erfinder: **Erdelen, Christoph, Dr.**
**Unterbüscherhof 22**
**D-42799 Leichlingen(DE)**
Erfinder: **Stendel, Wilhelm, Dr.**
**In den Birken 55**
**D-42113 Wuppertal(DE)**

(54) **Substituierte 4,5-Dihydro-1-pyrazolcarbonsäureanilide, Verfahren zu ihrer Herstellung und ihre Verwendung als Schädlingsbekämpfungsmittel.**

(57) Neue substituierte 4,5-Dihydro-1-pyrazolcarbonsäureanilide der allgemeinen Formel (I),

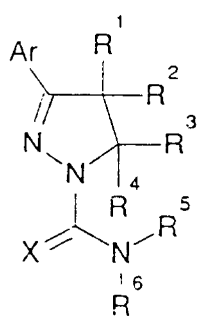

(I)

in welcher

$R^1$ für einen gegebenenfalls substituierten Heterocyclus aus der Reihe

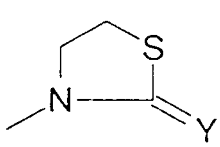

oder



Rank Xerox (UK) Business Services
(3.10/3.09/3.3.4)

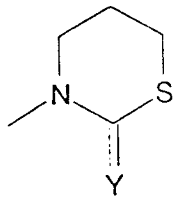

steht in welcher

Y für Sauerstoff oder Schwefel steht,

Verfahren zu ihrer Herstellung und ihre Verwendung als Schädlingsbekämpfungsmittel.

Die Erfindung betrifft neue substituierte 4,5-Dihydro-1-pyrazolcarbonsäureanilide, Verfahren zu ihrer Herstellung und ihre Verwendung als Schädlingsbekämpfungmittel.

Es ist bekannt, daß bestimmte substituierte Pyrazolinderivate eine gute Wirksamkeit gegen tierische Schädlinge besitzen.

Siehe dazu z.B. DE-A-2 700 258, US-A-4 174 393, DE-A-2 529 689, US-A-4 070 365 und EP-A-0 466 408, DE-A-4 001 931, DE-A-4 117 076.

Die Wirkungshöhe bzw. Wirkungsdauer dieser vorbekannten Verbindungen ist jedoch, insbesondere bei bestimmten Organismen oder bei niedrigen Anwendungskonzentrationen nicht in allen Anwendungsgebieten völlig zufriedenstellend.

Es wurden neue substituierte 4,5-Dihydro-1-pyrazolcarbonsäureanilide der allgemeinen Formel (I),

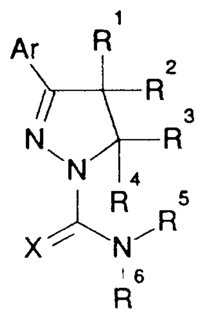

(I)

in welcher

| | |
|---|---|
| $R^1$ | für einen gegebenenfalls substituierten Heterocyclus aus der Reihe |

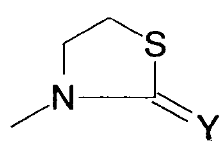

oder

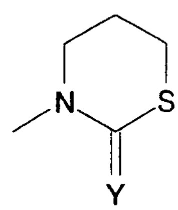

steht

in welcher

| | |
|---|---|
| Y | für Sauerstoff oder Schwefel steht, |
| $R^2$ | für Wasserstoff, Alkyl, gegebenenfalls substituiertes Cycloalkyl, Halogenalkyl, Halogenalkylthio oder Alkoxycarbonyl steht, |
| $R^3$ | für Wasserstoff, Alkyl oder für eine Gruppierung |

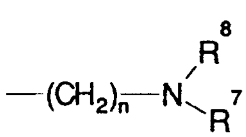

oder

$-(CH_2)_n-O-R^9$ steht

worin

| | |
|---|---|
| $R^7$ und $R^8$ | jeweils unabhängig voneinander für Wasserstoff, Alkyl oder Aryl stehen und |
| $R^9$ | für Wasserstoff, Alkyl oder Aryl steht und |
| n | für die Zahlen 1 bis 6 steht, |
| $R^4$ | für Wasserstoff oder Alkyl steht, |
| $R^5$ | für Wasserstoff, Alkyl, Phenyl oder Alkylthio steht, |
| $R^6$ | für gegebenenfalls substituiertes Alkyl, gegebenenfalls substituiertes Cycloalkyl oder für den Rest |

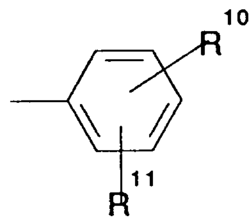

steht,

worin $R^{10}$ und $R^{11}$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, gegebenenfalls substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl stehen oder wobei $R^{10}$ und $R^{11}$ zusammen für einen bivalenten gegebenenfalls ein oder zwei Sauerstoffatome enthaltenden und gegebenenfalls substituierten Rest stehen,

X für Sauerstoff oder Schwefel steht und

Ar für gegebenenfalls substituiertes Aryl oder für einen gegebenenfalls substituierten und/oder gegebenenfalls anellierten Heterocyclus steht,

gefunden.

Weiterhin wurde gefunden, daß man die neuen 4,5-Dihydro-1-pyrazolcarbonsäureanilide der allgemeinem Formel (I)

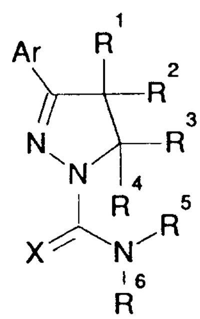

(I)

in welcher

$R^1$ fuhr einen gegebenenfalls substituierten Heterocyclus aus der Reihe

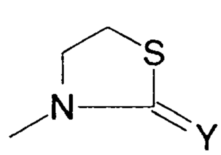 oder 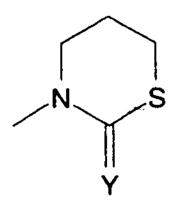

steht

in welcher

Y für Sauerstoff oder Schwefel steht,

$R^2$ für Wasserstoff, Alkyl, gegebenenfalls substituiertes Cycloalkyl, Halogenalkyl, Halogenalkylthio oder Alkoxycarbonyl steht,

$R^3$ für Wasserstoff, Alkyl oder für eine Gruppierung

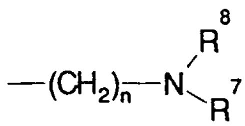

oder $-(CH_2)_n-O-R^9$ steht
worin

$R^7$ und $R^8$ jeweils unabhängig voneinander für Wasserstoff, Alkyl oder Aryl stehen und
$R^9$ für Wasserstoff, Alkyl oder Aryl steht und
n für die Zahlen 1 bis 6 steht,
$R^4$ für Wasserstoff oder Alkyl steht,
$R^5$ für Wasserstoff, Alkyl, Phenyl oder Alkylthio steht,
$R^6$ für gegebenenfalls substituiertes Alkyl, gegebenenfalls substituiertes Cycloalkyl oder für den Rest

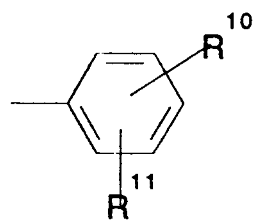

steht,

worin $R^{10}$ und $R^{11}$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, gegebenenfalls substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl stehen oder wobei $R^{10}$ und $R^{11}$ zusammen für einen bivalenten gegebenenfalls ein oder zwei Sauerstoffatome enthaltenden und gegebenenfalls substituierten Rest stehen,

X für Sauerstoff oder Schwefel steht und
Ar für gegebenenfalls substituiertes Aryl oder für einen gegebenenfalls substituierten und/oder gegebenenfalls anellierten Heterocyclus steht,

erhält, wenn man

(A) zum Erhalt von 4,5-Dihydro-1-pyrazolcarbonsäureaniliden der Formel (I), in welcher $R^5$ für Wasserstoff steht, Pyrazolinderivate der Formel (II)

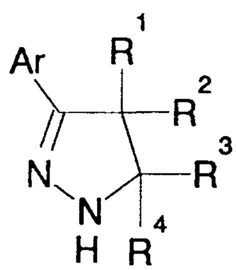

(II)

in welcher

$R^1$, $R^2$, $R^3$, $R^4$ und Ar die oben angegebene Bedeutung besitzen, mit Isocyanaten bzw. Isothiocyanaten der Formel (III)

$X=C=N-R^6$ (III)

in welcher

X und $R^6$ die oben angegebene Bedeutung haben,
gegebenenfalls in Gegenwart von Basen umsetzt oder wenn man
(B) zum Erhalt von Pyrazolinderivaten der Formel (I), in welcher $R^2$ für Alkyl, Cycloalkyl, Halogenalkyl, Halogenalkylthio oder Alkoxycarbonyl steht, Verbindungen der Formel (IV)

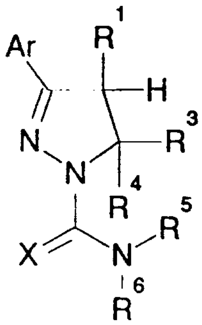

(IV)

mit Verbindungen der Formel (V)

$Hal\text{-}R^{2-1}$ (V)

in welcher

Hal für Halogen steht und
$R^{2-1}$ für Alkyl, gegebenenfalls substituiertes Cycloalkyl, Halogenalkyl, Halogenalkylthio oder Alkoxycarbonyl steht,

in wasserfreiem Medium unter Zusatz einer starken Base umsetzt.

Schließlich wurde gefunden, daß die neuen 4,5-Dihydro-1-pyrazolcarbonsäureanilide der allgemeinen Formel (I) eine sehr gute Wirkung gegen Schädlinge und insbesondere eine sehr gute insektizide und akarizide Wirksamkeit besitzen.

Überraschenderweise zeigen die erfindungsgemäßen substituierten 4,5-Dihydro-1-pyrazolcarbonsäureanilide eine erheblich bessere insektizide Wirksamkeit gegen pflanzenschädigende und warmblüterparasitierende Insekten und Spinnentiere als aus dem Stand der Technik bekannte chemisch und wirkungsmäßig naheliegende Verbindungen.

Die erfindungsgemäßen substituierten 4,5-Dihydro-1-pyrazolcarbonsäureanilidesind durch die Formel (I) allgemein definiert Bevorzugt sind Verbindungen der Formel (I), bei welchen

$R^1$ für einen unsubstituierten oder einfach bis mehrfach, gleich oder verschieden substituierten Heterocyclus aus der Reihe

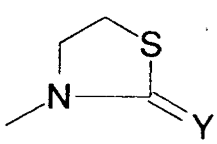

oder

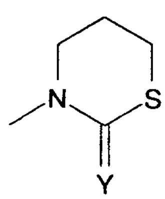

steht
in welcher

Y für Sauerstoff oder Schwefel steht,

und wobei folgende Substituenten infrage kommen: Alkyl ($C_1$-$C_6$), Alkoxy ($C_1$-$C_6$), Alkyl($C_1$-$C_6$)thio, Amino, Alkyl($C_1$-$C_6$)amino, Dialkyl($C_1$-$C_6$)amino und gegebenenfalls durch Halogen, Alkyl($C_1$-$C_4$), Alkoxy($C_1$-$C_4$), Halogenalkyl($C_1$-$C_4$), Halogenalkoxy($C_1$-$C_4$)- oder Halogenalkyl($C_1$-$C_4$)thio substituiertes Phenyl,

$R^2$ für Wasserstoff, Alkyl($C_1$-$C_6$), gegebenenfalls durch Halogen, Halogenalkyl($C_1$-$C_4$) substituiertes Cycloalkyl($C_3$-$C_7$); Halogenalkyl($C_1$-$C_4$)thio oder Alkoxy($C_1$-$C_6$)carbonyl steht,

$R^3$ für Wasserstoff, Alkyl($C_1$-$C_6$) oder für eine Gruppierung

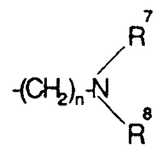

oder $-(CH_2)_n-OR^9$ steht,
worin

$R^7$ und $R^8$ jeweils unabhängig voneinander für Wasserstoff, Alkyl($C_1$-$C_6$) oder für gegebenenfalls durch Alkyl($C_1$-$C_4$), Alkoxy($C_1$-$C_4$), Alkyl($C_1$-$C_4$)thio, Halogen, Halogenalkyl($C_1$-$C_4$), Halogenalkoxy($C_1$-$C_4$) oder Halogenalkyl($C_1$-$C_4$)thio substituiertes Phenyl stehen und

$R^9$ für Wasserstoff, Alkyl($C_1$-$C_6$) oder gegebenenfalls substituiertes Phenyl steht, wobei als Substituenten die für $R^8$ aufgeführten Phenylsubstituenten infrage kommen und

n für die Zahlen 1 bis 4 steht,

$R^4$ für Wasserstoff oder Alkyl($C_1$-$C_6$) steht,

$R^5$ für Wasserstoff, Alkyl($C_1$-$C_6$), Phenyl oder Alkyl($C_1$-$C_4$)thio steht,

$R^6$ für gegebenenfalls durch Halogen, Halogenalkyl($C_1$-$C_4$), Halogenalkoxy($C_1$-$C_4$) substituiertes Alkyl($C_1$-$C_6$),
für gegebenenfalls durch Halogen, Halogenalkyl($C_1$-$C_4$),Halogenalkoxy($C_1$-$C_4$) substituiertes Cycloalkyl($C_3$-$C_7$) oder für den Rest

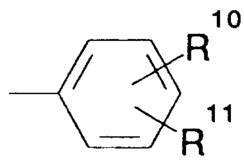

steht,
wobei

$R^{10}$ und $R^{11}$ gleich oder verschieden sein können und für Halogen, Alkyl($C_1$-$C_6$), Nitro, Cyano, Halogenalkyl($C_1$-$C_4$), Alkoxy($C_1$-$C_6$), Halogenalkoxy($C_1$-$C_4$), Alkyl($C_1$-$C_4$)thio, Halogenalkyl($C_1$-$C_4$)thio, gegebenenfalls durch Halogen, Halogenalkyl($C_1$-$C_4$), Alkoxy($C_1$-$C_4$), Alkyl($C_1$-$C_4$) substituiertes Phenoxy oder Phenylthio, gegebenenfalls durch Halogen, Alkoxy($C_1$-$C_4$), Halogenalkyl($C_1$-$C_4$) substituiertes Mono- oder Dialkylamino mit jeweils 1 bis 6 Kohlenstoffatomen im Alkylrest, gegebenenfalls durch Alkyl($C_1$-$C_4$), Alkoxy($C_1$-$C_4$), Halogen, Alkyl($C_1$-$C_4$)thio substituiertes Cycloalkyl($C_3$-$C_7$), Alkoxy($C_1$-$C_4$)carbonyl, Alkenyl($C_2$-$C_6$)oxy, Alkinyl($C_2$-$C_6$), Alkyl($C_1$-$C_4$)thionyl, Alkyl($C_1$-$C_4$)sulfonyl, Halogenalkyl($C_1$-$C_4$)thionyl, Halogenalkyl($C_1$-$C_4$)sulfonyl, Halogenalkoxy($C_1$-$C_4$)-carbonyl stehen oder wobei

$R^{10}$ und $R^{11}$ zsammen für einen der folgenden bivalenten Reste stehen

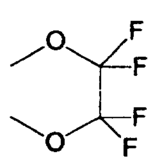 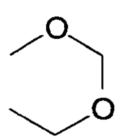 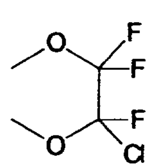 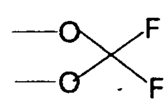

X für Sauerstoff oder Schwefel steht und

Ar für gegebenenfalls substituiertes Phenyl oder Naphthyl steht, wobei als Substituenten genannt seien:
Alkyl, Halogen, Halogenalkyl, Alkoxy, Alkylthio, Halogenalkoxy, Halogenalkylthio, Alkoxycarbonyl, Halogenalkoxycarbonyl, gegebenenfalls substituiertes Aryloxy, gegebenenfalls substituiertes Arylthio, Alkenyloxy, Alkinyl, Alkylthionyl, Alkylsulfonyl, Halogenalkylthionyl, Halogenalkylsulfonyl, Nitro, Cyano oder wobei zwei benachbarte Positionen durch einen durch Halogen substituierten 3,4-Methylendioxyl- oder 3,4-Ethylendioxylrest miteinander

verbunden sind;

weiterhin steht Ar für einen unsubstituierten oder einfach bis mehrfach, gleich oder verschieden substituierten, gegebenenfalls benzoanellierten heteroaromatischen 5- oder 6-gliedrigen Ring, der gleiche oder verschiedene, ein oder mehrere Heteroatome wie Sauerstoff, Schwefel und Stickstoff enthält, wobei als Substituenten die oben für Phenyl und Naphthyl aufgeführten Substituenten infrage kommen.

Besonders bevorzugt sind die Verbindungen der Formel (I), bei welchen

$R^1$ für einen unsubstituierten oder einfach bis dreifach, gleich oder verschieden substituierten Heterocyclus aus der Reihe

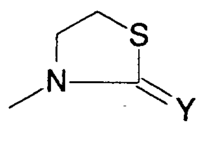 oder 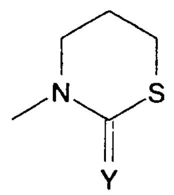

steht,
in welcher

Y für Sauerstoff oder Schwefel steht,
und wobei die folgenden Substituenten infrage kommen:
Alkyl($C_1$-$C_4$), Alkoxy($C_1$-$C_4$), Alkyl($C_1$-$C_4$)thio, Amino, Alkyl($C_1$-$C_4$)amino, Dialkyl($C_1$-$C_4$)-amino und gegebenenfalls durch Fluor, Chlor, Brom, Methyl, Ethyl, Methoxy, Ethoxy, Halogenalkyl($C_1$-$C_2$),Halogenalkoxy($C_1$-$C_2$) oder Halogenalkyl($C_1$-$C_2$)thio substituiertes Phenyl,

$R^2$ für Wasserstoff, Alkyl($C_1$-$C_4$), gegebenenfalls durch Fluor, Chlor, Brom, Halogenalkyl($C_1$-$C_3$) substituiertes Cycloalkyl($C_3$-$C_6$), Halogenalkyl($C_1$-$C_3$), Halogenalkyl($C_1$-$C_3$)thio oder Alkoxy($C_1$-$C_4$)carbonyl steht,

$R^3$ für Wasserstoff, Alkyl($C_1$-$C_4$) oder für eine Gruppierung

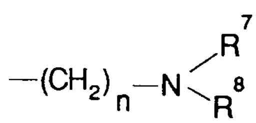

oder $-(CH_2)_n-OR^9$ steht,
worin

$R^7$ und $R^8$ jeweils unabhängig voneinander für Wasserstoff, Alkyl($C_1$-$C_4$) oder für gegebenenfalls durch Methyl, Ethyl, Methoxy, Ethoxy, Methylthio, Ethylthio, Fluor, Chlor, Brom, Halogenalkyl($C_1$-$C_2$), Halogenalkoxy($C_1$-$C_2$) oder Halogenalkyl($C_1$-$C_2$)thio substituiertes Phenyl stehen und

$R^9$ für Wasserstoff, Alkyl($C_1$-$C_4$) oder gegebenenfalls einfach oder mehrfach, gleich oder verschieden substituiertes Phenyl steht, wobei als Substituenten die für $R^8$ aufgeführten Phenylsubstituenten infrage kommen und

n für die Zahlen 1,2 oder 3 steht,

$R^4$ für Wasserstoff oder Alkyl($C_1$-$C_4$) steht,

$R^5$ für Wasserstoff, Alkyl($C_1$-$C_4$), Phenyl oder Alkyl($C_1$-$C_3$)thio steht,

$R^6$ für gegebenenfalls durch Fluor, Chlor, Brom, Halogenalkyl($C_1$-$C_3$), Halogenalkoxy($C_1$-$C_3$) substituiertes Alkyl($C_1$-$C_4$), für gegebenenfalls durch Fluor, Chlor, Brom, Halogenalkyl($C_1$-$C_3$), Halogenalkoxy($C_1$-$C_3$) substituiertes Cycloalkyl($C_3$-$C_6$) oder für den Rest

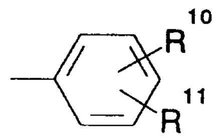

steht,

wobei

$R^{10}$ und $R^{11}$ gleich oder verschieden sein können und für Fluor, Chlor, Brom, Iod, Alkyl($C_1$-$C_4$), Nitro, Cyano, Halogenalkyl($C_1$-$C_3$), Alkoxy($C_1$-$C_4$), Halogenalkoxy($C_1$-$C_3$), Alkyl($C_1$-$C_3$)thio, Halogenalkyl($C_1$-$C_3$)thio, gegebenenfalls durch Fluor, Chlor, Brom, Halogenalkyl($C_1$-$C_3$)-,Alkoxy($C_1$-$C_3$), Alkyl($C_1$-$C_3$) substituiertes Phenoxy, gegebenenfalls durch Fluor, Chlor, Brom, Alkoxy($C_1$-$C_3$), Halogenalkyl($C_1$-$C_3$) substituiertes Mono- oder Dialkylamino mit jeweils 1 bis 4 Kohlenstoffatomen im Alkylrest gegebenenfalls durch Alkyl($C_1$-$C_3$), Alkoxy($C_1$-$C_3$), Fluor, Chlor, Brom, Alkyl($C_1$-$C_3$)thio substituiertes Cycloalkyl($C_3$-$C_6$) stehen oder wobei

$R^{10}$ und $R^{11}$ zusammen für einen der folgenden bivalenten Reste stehen:

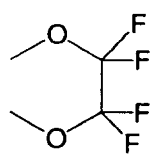 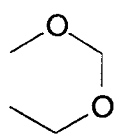 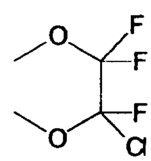 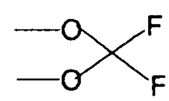

X für Sauerstoff oder Schwefel steht und

Ar für gegebenenfalls einfach bis dreifach, gleich oder verschieden substituiertes Phenyl oder für einen unsubstituierten oder einfach bis dreifach, gleich oder verschieden substituierten heteroaromatischen 5- oder 6-gliedrigen Ring steht, der gleiche oder verschiedene, ein oder mehrere Heteroatome wie Sauerstoff, Schwefel oder Stickstoff enthält, wobei als Substituenten genannt seien:

Alkyl($C_1$-$C_4$), Fluor, Chlor, Brom, Halogenalkyl($C_1$-$C_4$), Alkoxy($C_1$-$C_4$), Alkyl($C_1$-$C_4$)thio, Halogenalkoxy($C_1$-$C_3$), Halogenalkyl($C_1$-$C_3$)thio, Alkoxy($C_1$-$C_3$)carbonyl, gegebenenfalls durch Fluor, Chlor, Brom, Alkyl($C_1$-$C_3$),Alkoxy($C_1$-$C_3$), Halogenalkyl($C_1$-$C_3$) substituiertes Phenoxy oder Phenylthio, Alkenyl($C_2$-$C_4$)oxy, Alkinyl($C_2$-$C_4$), Alkyl($C_1$-$C_3$)thionyl, Alkyl-($C_1$-$C_3$)sulfonyl, Halogenalkyl($C_1$-$C_3$)thionyl, Halogenalkyl($C_1$-$C_3$)sulfonyl, Nitro, Cyano stehen oder

wobei zwei benachbarte Positionen durch einen durch Fluor und/oder Chlor substituierten 3,4-Methylendioxy- oder 3,4-Ethylendioxyrest miteinander verbunden sind.

Insbesondere bevorzugt sind Verbindungen der Formel (I) bei welchen

$R^1$ für einen unsubstituierten oder einfach bis dreifach, gleich oder verschieden substituierten Heterocyclus aus der Reihe

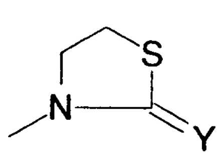 oder 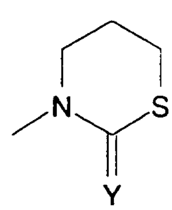

steht,

in welcher

Y für Sauerstoff oder Schwefel steht und

wobei die folgenden Substituenten infrage kommen:

Methyl, Ethyl, n-, i-Propyl, t-Butyl und gegebenenfalls durch Fluor, Chlor, Brom, Methyl,

Methoxy, Trifluormethyl, Trifluormethoxy oder Trifluormethylthio substituiertes Phenyl,

$R^2$ für Wasserstoff, Methyl, Ethyl, n-Propyl, i-Propyl oder Alkoxy($C_1$-$C_2$)carbonyl steht,

$R^3$ für Wasserstoff, Methyl, Ethyl, n-Propyl, i-Propyl oder für die Gruppe

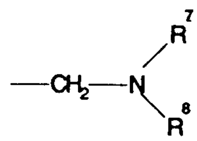

steht,

worin

$R^7$ und $R^8$ jeweils unabhängig voneinander für Wasserstoff, Methyl, Ethyl, n-Propyl, i-Propyl steht,

$R^4$ für Wasserstoff, Methyl, Ethyl oder n-Propyl steht,

$R^5$ für Wasserstoff, Methyl, Ethyl, n-Propyl, i-Propyl, Phenyl oder Alkyl($C_1$-$C_2$)thio steht,

$R^6$ für gegebenenfalls durch Fluor, Chlor, Halogenalkyl($C_1$-$C_3$), Halogenalkoxy($C_1$-$C_3$) substituiertes Methyl, Ethyl, n-Propyl oder i-Propyl für gegebenenfalls durch Fluor, Chlor, Halogenalkyl($C_1$-$C_3$), Halogenalkoxy($C_1$-$C_3$) substituiertes Cycloalkyl ($C_3$-$C_6$) oder für den Rest

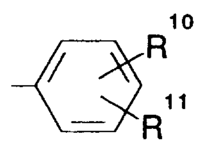

steht,

wobei

$R^{10}$ und $R^{11}$ gleich oder verschieden sein können und für Fluor, Chlor, Brom, Iod, Methyl, Ethyl, n-Propyl, i-Propyl, tert-Butyl, Nitro, Cyano, Halogenalkyl($C_1$-$C_3$), Alkoxy($C_1$-$C_3$), Halogenalkoxy($C_1$-$C_3$), Alkyl($C_1$-$C_3$)thio, Halogenalkyl($C_1$-$C_3$)thio, gegebenenfalls durch Fluor, Chlor, Halogenalkyl($C_1$-$C_3$), Methoxy, Ethoxy, Methyl, Ethyl substituiertes Phenoxy, gegebenenfalls durch Fluor, Chlor, Methoxy, Ethoxy, Halogenalkyl($C_1$-$C_3$) substituiertes Mono- oder Dialkylamino mit jeweils 1 bis 3 Kohlenstoffatomen im Alkylrest, gegebenenfalls durch Methyl, Ethyl, Methoxy, Ethoxy, Fluor, Chlor, Alkyl($C_1$-$C_3$)thio substituiertes Cycloalkyl($C_3$-$C_6$) stehen oder wobei

$R^{10}$ und $R^{11}$ zusammen für einen der folgenden bivalenten Reste stehen

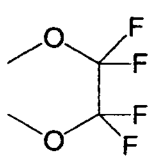

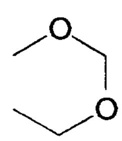

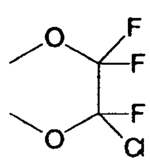

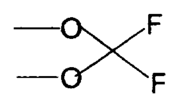

X für Sauerstoff oder Schwefel steht und

Ar für gegebenenfalls einfach bis dreifach, gleich oder verschieden substituiertes Phenyl oder für einen jeweils unsubstituierten oder einfach bis dreifach, gleich oder verschieden substituierten Pyridyl-, Pyrazinyl-, Thiazolyl-, Pyrimidyl-, Pyridazinyl, Thienyl-, Furyl- oder 1,3-Benzodioxyl-Rest steht, wobei als Substituenten genannt seien:
Methyl, Ethyl, n-Propyl, i-Propyl, Fluor, Chlor, Halogenalkyl($C_1$-$C_3$), Methoxy, Ethoxy, n-Propyloxy, i-Propyloxy, Alkyl($C_1$-$C_3$)thio, Halogenalkoxy($C_1$-$C_3$), Halogenalkyl($C_1$-$C_3$)thio, Alkoxy($C_1$-$C_3$)carbonyl, gegebenenfalls durch Fluor, Chlor, Methyl, Ethyl, Methoxy, Ethoxy, Halogenalkyl($C_1$-$C_3$) substituiertes Phenoxy oder Phenylthio, Alkenyl($C_3$-$C_4$)oxy, Alkinyl($C_2$-$C_4$), Alkyl($C_1$-$C_3$)thionyl, Alkyl($C_1$-$C_3$)sulfonyl, Halogenalkyl($C_1$-$C_3$)thionyl, Halogenalkyl($C_1$-$C_3$)sulfonyl, Nitro, Cyano stehen oder
wobei zwei benachbarte Positionen durch einen durch Fluor und/oder Chlor substituierten 3,4-Methylendioxy- oder 3,4-Ethylendioxyrest miteinander verbunden sind.

Die Substituentenbedeutung Halogenalkyl in den Resten Halogenalkyl, Halogenalkoxy, Halogenalkylthio, Halogenalkylthionyl und Halogenalkylsulfonyl enthält vorzugsweise 1 bis 4, insbesondere 1 oder 2 Kohlenstoffatome und vorzugsweise 1 bis 5, insbesondere 1 bis 3 gleiche oder verschiedene Halogenatome, wobei als Halogenatome vorzugsweise Fluor, Chlor und Brom, insbesondere Fluor und Chlor stehen. Beispielhaft seien Trifluormethyl, Chlordifluormethyl, Brommethyl, 2,2,2-Trifluorethyl und Pentafluorethyl genannt.

Im einzelnen seien außer den bei den Herstellungsbeispielen genannten Verbindungen die folgenden, beispielhaft genannten substituierten 4,5-Dihydro-1-pyrazolcarbonsäureanilide der allgemeinen Formel (I) genannt:

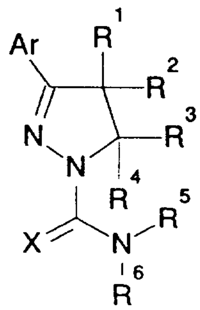

(I)

Tabelle 1:

| Ar | $R^2$ | $R^3$ | $R^4$ | $R^5$ | $R^6$ | X | $R^1$ |
|---|---|---|---|---|---|---|---|
| 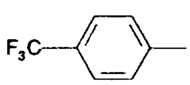 | H | H | H | H | 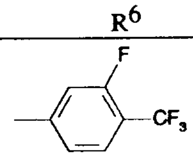 | O | 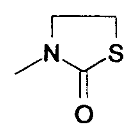 |
| 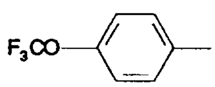 | H | H | H | H | 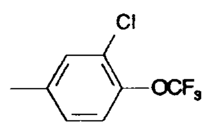 | O | 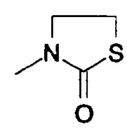 |
| 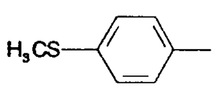 | H | H | H | H | 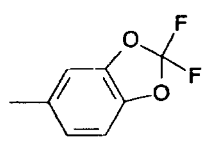 | O | 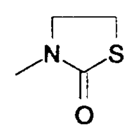 |
| 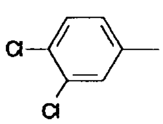 | H | H | H | H | 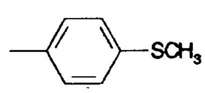 | O | 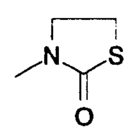 |
| 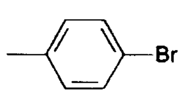 | H | H | H | H | 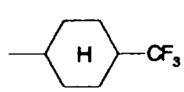 | O | 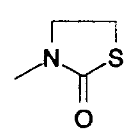 |
| 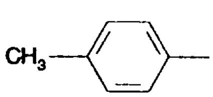 | H | H | H | H | 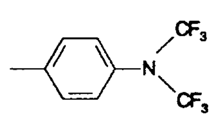 | O | 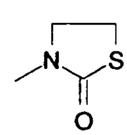 |

| Ar | $R^2$ | $R^3$ | $R^4$ | $R^5$ | $R^6$ | X | $R^1$ |
|---|---|---|---|---|---|---|---|
| 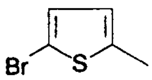 | H | H | H | H | 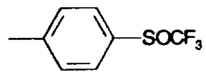 | O | 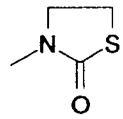 |
| 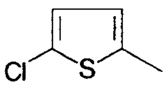 | H | H | H | H | 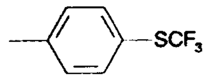 | O | 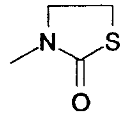 |
| 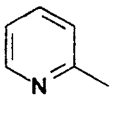 | H | H | H | H | 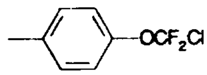 | O | 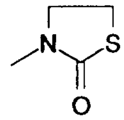 |
| 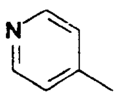 | H | H | H | H | 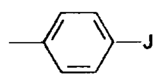 | O | 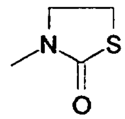 |
| 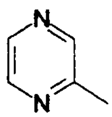 | H | H | H | H | 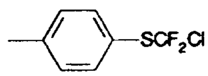 | O | 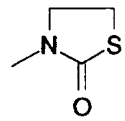 |
| 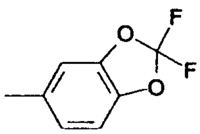 | H | H | H | H | 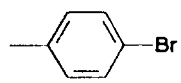 | O | 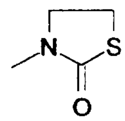 |
| 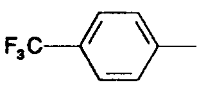 | H | H | H | H | 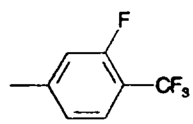 | O | 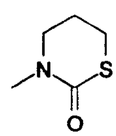 |

| Ar | $R^2$ | $R^3$ | $R^4$ | $R^5$ | $R^6$ | X | $R^1$ |
|---|---|---|---|---|---|---|---|
| 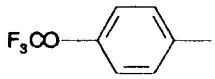 | H | H | H | H | 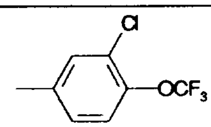 | O | 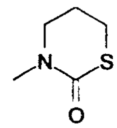 |
| 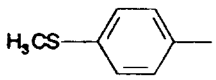 | H | H | H | H | 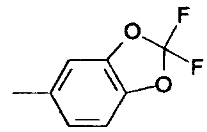 | O | 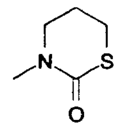 |
| 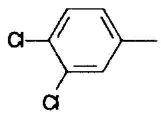 | H | H | H | H | 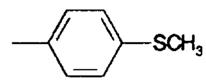 | O | 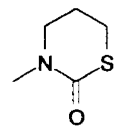 |
| 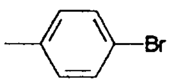 | H | H | H | H | 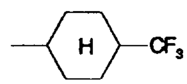 | O | 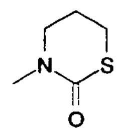 |
| 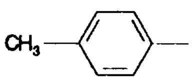 | H | H | H | H | 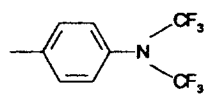 | O | 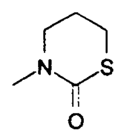 |
| 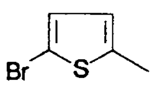 | H | H | H | H | 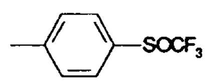 | O | 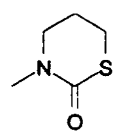 |
| 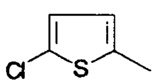 | H | H | H | H | 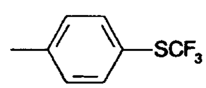 | O | 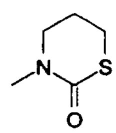 |

| Ar | $R^2$ | $R^3$ | $R^4$ | $R^5$ | $R^6$ | X | $R^1$ |
|---|---|---|---|---|---|---|---|
| 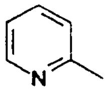 | H | H | H | H | 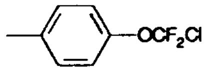 | O | 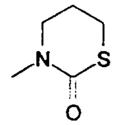 |
| 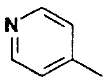 | H | H | H | H | 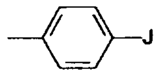 | O | 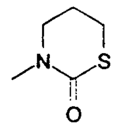 |
| 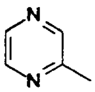 | H | H | H | H | 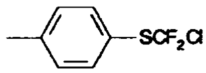 | O | 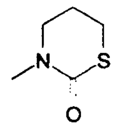 |
| 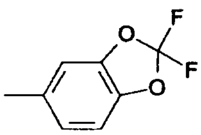 | H | H | H | H | 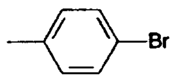 | O | 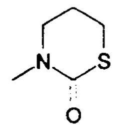 |
| 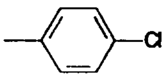 | $CH_3$ | H | H | H | 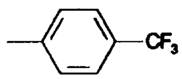 | O | 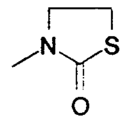 |
| 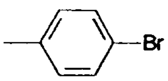 | 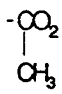 | H | H | H | 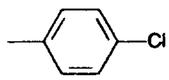 | O | 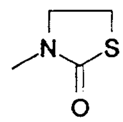 |
| 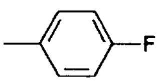 | H | $CH_3$ | H | H | 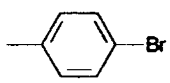 | O | 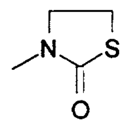 |

| Ar | $R^2$ | $R^3$ | $R^4$ | $R^5$ | $R^6$ | X | $R^1$ |
|---|---|---|---|---|---|---|---|
| 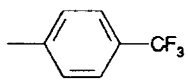 | H | $CH_3$ | $CH_3$ | H | 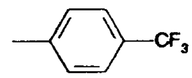 | O | 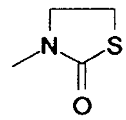 |
| 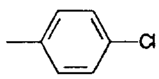 | H | H | H | H | 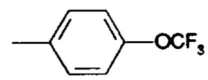 | S | 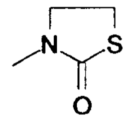 |
| 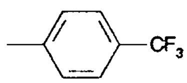 | H | 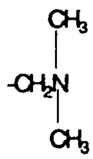 | H | H | 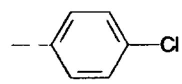 | O | 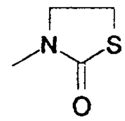 |
| 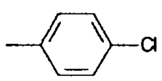 | H | 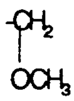 | H | H | 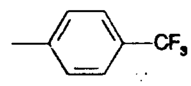 | O | 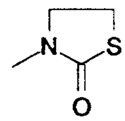 |
| 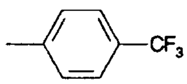 | H | 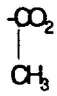 | H | H | 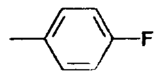 | O | 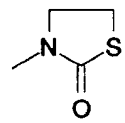 |
| 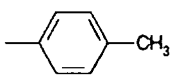 | 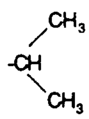 | H | H | H | 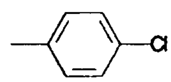 | O | 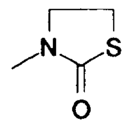 |
| 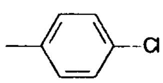 | H | 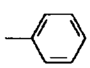 | H | H | 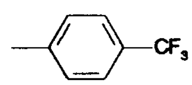 | O | 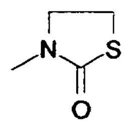 |

| Ar | $R^2$ | $R^3$ | $R^4$ | $R^5$ | $R^6$ | X | $R^1$ |
|---|---|---|---|---|---|---|---|
| 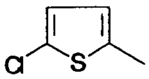 | H | H | 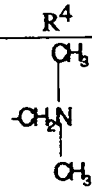 | H | 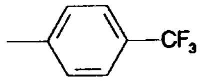 | O | 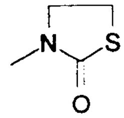 |
| 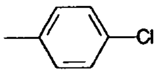 | H | H | H | H | 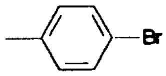 | O | 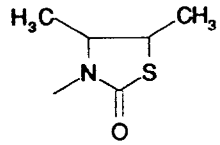 |
| 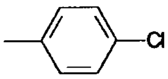 | $CH_3$ | H | H | H | 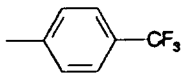 | O | 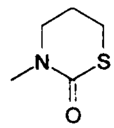 |
| 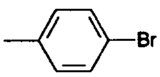 | 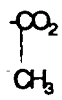 | H | H | H | 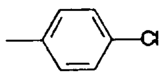 | O | 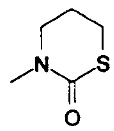 |
| 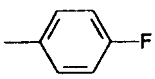 | H | $CH_3$ | H | H | 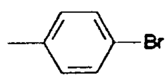 | O | 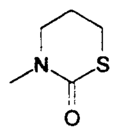 |
| 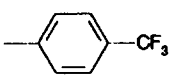 | H | $CH_3$ | $CH_3$ | H | 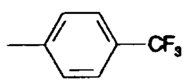 | O | 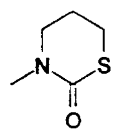 |

| Ar | $R^2$ | $R^3$ | $R^4$ | $R^5$ | $R^6$ | X | $R^1$ |
|---|---|---|---|---|---|---|---|
| 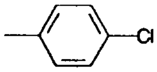 | H | H | H | H | 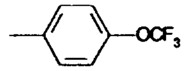 | S | 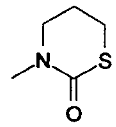 |
| 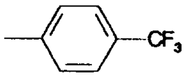 | H | 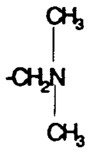 | H | H | 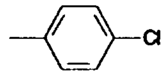 | O | 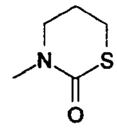 |
| 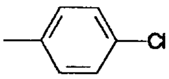 | H | 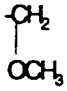 | H | H | 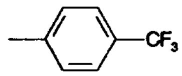 | O | 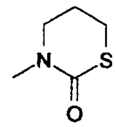 |
| 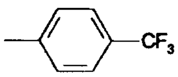 | H | 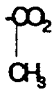 | H | H | 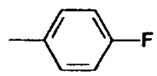 | O | 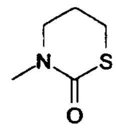 |
| 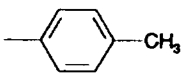 | 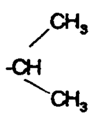 | H | H | H | 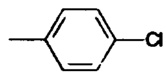 | O | 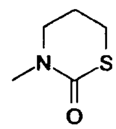 |
| 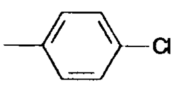 | H | 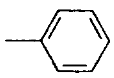 | H | H | 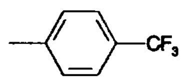 | O | 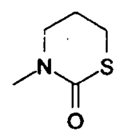 |
| 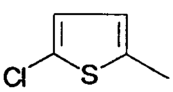 | H | H | 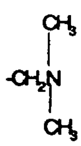 | H | 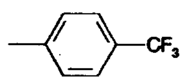 | O | 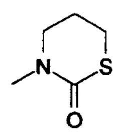 |

| Ar | $R^2$ | $R^3$ | $R^4$ | $R^5$ | $R^6$ | X | $R^1$ |
|---|---|---|---|---|---|---|---|
| 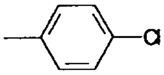 | $CH_3$ | H | H | H | 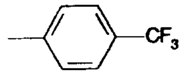 | O | 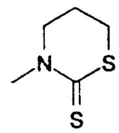 |
| 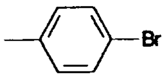 | 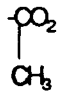 | H | H | H | 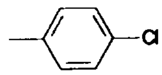 | O | 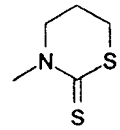 |
| 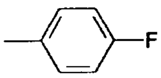 | H | $CH_3$ | H | H | 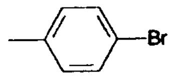 | O | 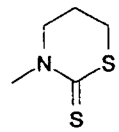 |
| 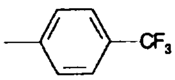 | H | $CH_3$ | $CH_3$ | H | 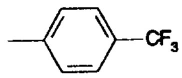 | O | 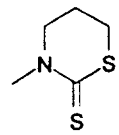 |
| 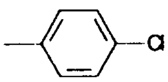 | H | H | H | H | 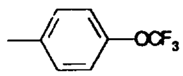 | S | 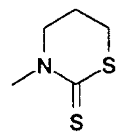 |
| 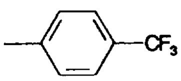 | H | 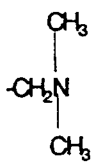 | H | H | 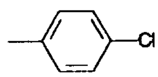 | O | 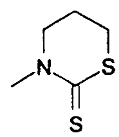 |
| 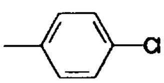 | H | 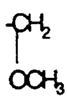 | H | H | 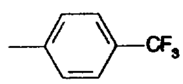 | O | 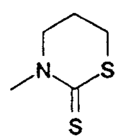 |

| Ar | $R^2$ | $R^3$ | $R^4$ | $R^5$ | $R^6$ | X | $R^1$ |
|---|---|---|---|---|---|---|---|
| 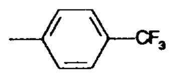 | H | 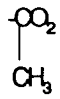 | H | H | 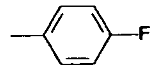 | O | 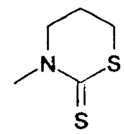 |
| 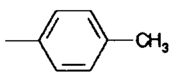 | 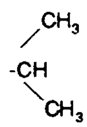 | H | H | H | 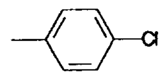 | O | 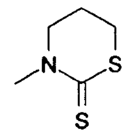 |
| 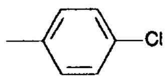 | H | 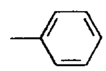 | H | H | 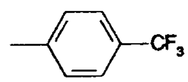 | O | 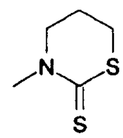 |
| 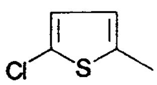 | H | H | 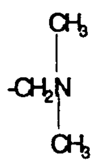 | H | 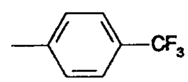 | O | 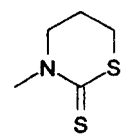 |
| 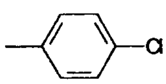 | H | H | H | H | 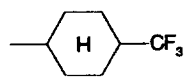 | O | 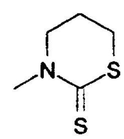 |
| 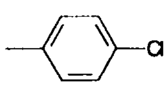 | H | H | H | H | 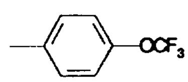 | O | 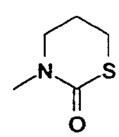 |
| 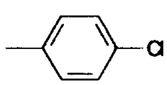 | H | H | H | H | 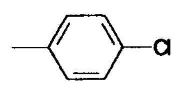 | O | 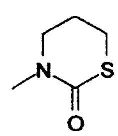 |

| Ar | $R^2$ | $R^3$ | $R^4$ | $R^5$ | $R^6$ | X | $R^1$ |
|---|---|---|---|---|---|---|---|
| 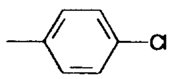 | H | H | H | H | 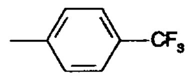 | O | 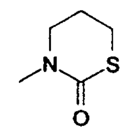 |
| 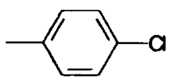 | H | H | H | H | 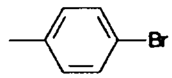 | O | 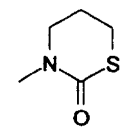 |
| 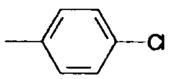 | H | H | H | H | 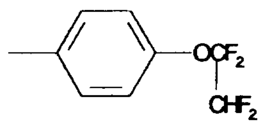 | O | 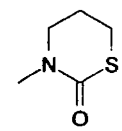 |

Verwendet man beispielsweise 3-(4'-Chlorphenyl)-4-(thiazolin-2''-on-3''-yl)-4,5-dihydropyrazol und 4-Trifluormethoxy-phenylisocyanat als Ausgangsstoffe, so läßt sich der Reaktionsablauf des erfindungsgemäßen Verfahrens A) durch das folgende Formelschema darstellen:

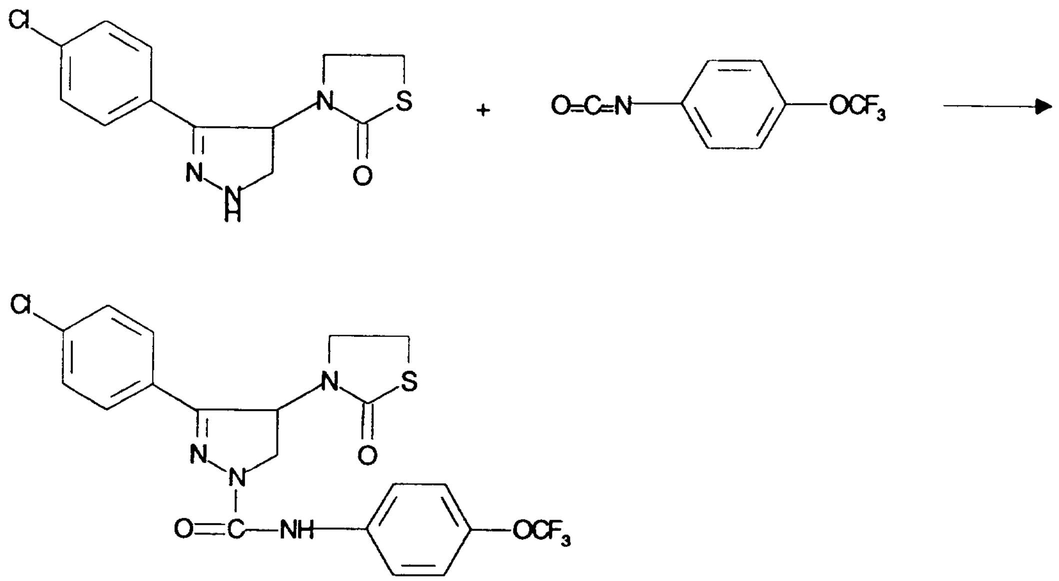

Verwendet man beispielsweise 3-(4'-Methylphenyl)-4-thiazolidin-2''-on-3''-yl)-4,5-dihydro-1-pyrazolcarbonsäure-(4-chioranilid) und 2-lodpropan als Ausgangsstoffe, so kann der Reaktionsablauf des erfindungsgemäßen Verfahrens B) wie folgt dargestellt werden:

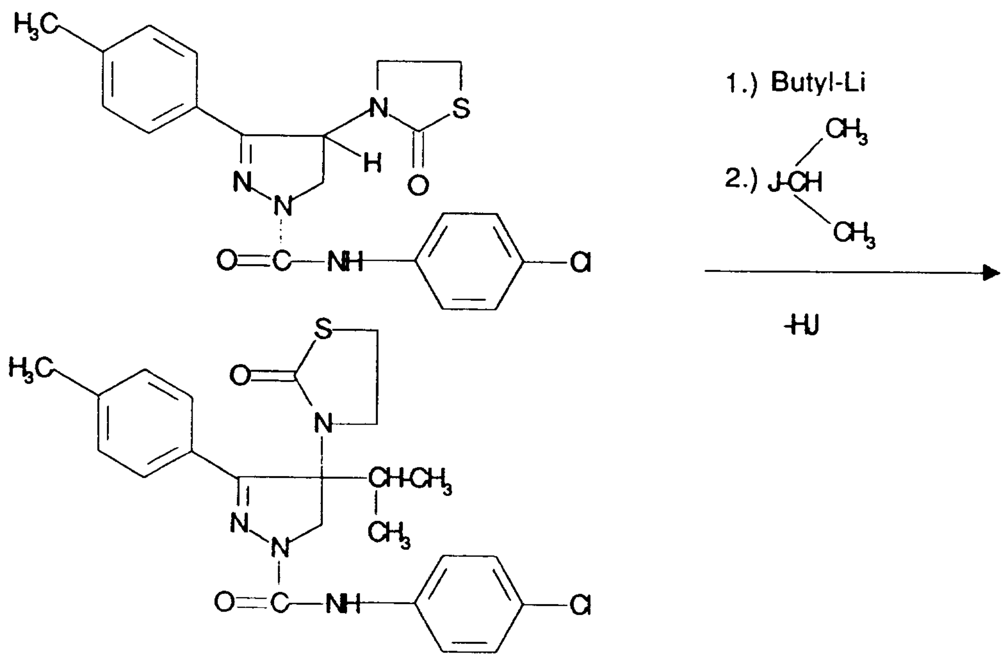


Die zur Durchführung des erfindungsgemäßen Verfahrens (A) als Ausgangsstoffe benötigten Pyrazolinderivate sind durch die Formel (II) allgemein definiert. In dieser Formel (II) stehen $R^1$, $R^2$, $R^3$, $R^4$ und Ar vorzugsweise für diejenigen Reste, die bereits im Zusammenhang mit der Beschreibung der erfindungsgemäßen Stoffe der Formel (I) als bevorzugt für diese Substituenten genannt wurden. Die Pyrazolinderivate der Formel (II) sind neu und können nach einem der folgenden Verfahren hergestellt werden: Sie werden durch Umsetzung von Verbindungen der Formel (VI)

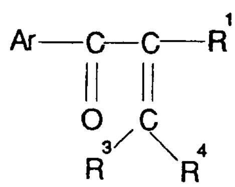
(VI)

in einem polaren organischen Lösungsmittel, vorzugsweise einem Alkanol bei Temperaturen von 20 bis 80 °C insbesondere bei 30 bis 60 °C mit Hydrazin-Hydrat hergestellt:

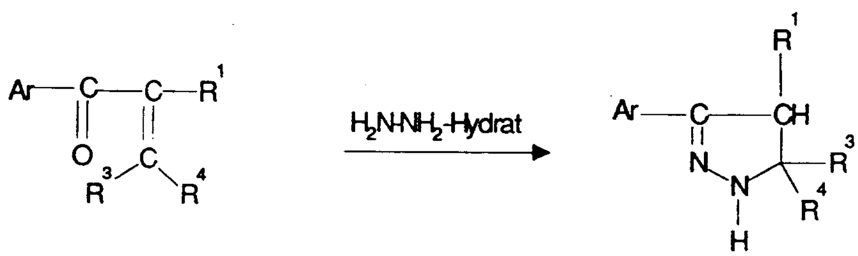


(VI) (II)

In Abhängigkeit von der Bedeutung der Substituenten $R^3$ und $R^4$ ergeben sich dabei folgende Herstellungsvarianten der Ausgangsverbindungen der Formel (VI)

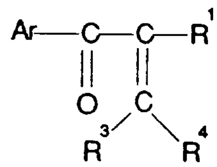

(VI)

(a) $R^3$ und $R^4$ in der Formel (VI) stehen fuhr Wasserstoff

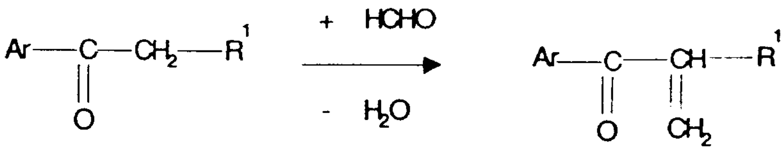


(VIa) (VIb)

Hierbei wird in einem polaren organischen Lösungsmittel, vorzugsweise einem Alkanol und insbesondere in Ethanol oder Methanol mit einer Formalinlösung unter Zusatz geringer Mengen einer organischen Base, insbesondere von Piperidin sowie Zusatz von Eisessig umgesetzt.

(b) In der Formel (VI) steht $R^3$ für Alkyl oder Aryl und $R^4$ steht für Wasserstoff

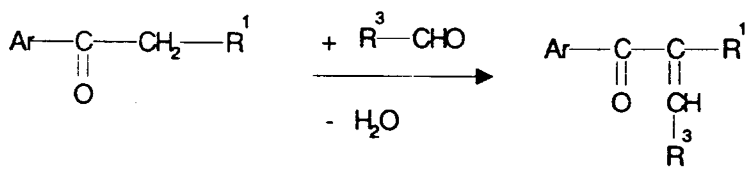


(VIa) (VIc)

Die Verfahrensbedingungen entsprechen denjenigen der Umsetzung mit Formaldehyd.

(c) In der Formel (VI) stehen $R^3$ und $R^4$ für Alkyl:

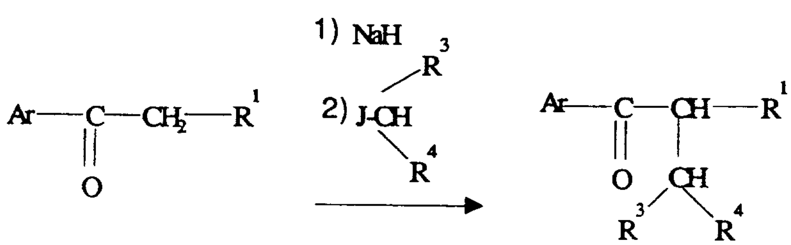


(VIa) (VII)

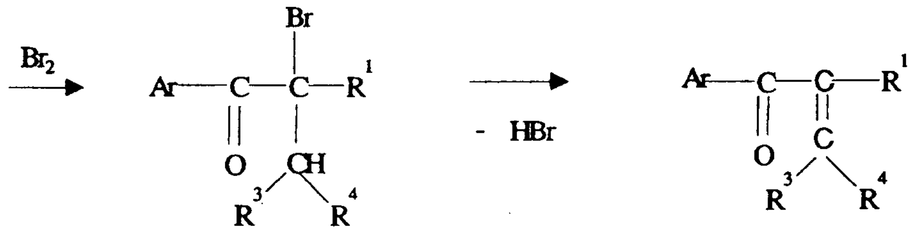


(VIII)

(IX)

Hierbei wird die Verbindung (VI) zunächst mit einer starken Base in das Salz übergeführt und anschließend mit einem Halogenid, insbesondere einem Jodid der Formel

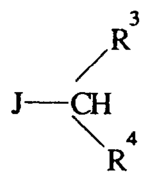

umgesetzt.

Die dabei entstehende Verbindung der Formel (VII) wird bromiert und anschließend wird durch Zusatz einer Base unter HBr-Eliminierung das Zwischenprodukt der Formel (IX) hergestellt.

Verbindungen der Formel (IIa), in welcher $R^3$ und $R^4$ für Wasserstoff stehen, werden außerdem durch Umsetzung von Verbindungen der Formel (VId)

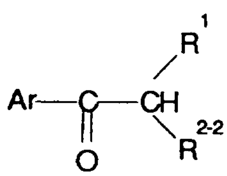

(VId)

in welcher

Ar und $R^1$ die oben angegebene Bedeutung haben und

$R^{2-2}$ für Wasserstoff, Alkyl oder Alkoxycarbonyl steht,

erhalten, indem man diese zunächst in einer 1. Stufe in einem polaren organischen Lösungsmittel, vorzugsweise Acetonitril, bei Temperaturen von 10 bis 100 °C, insbesondere bei 20 bis 80 °C mit einem Mol N,N-Dimethylmethylenimmoniumchlorid der Formel (XI)

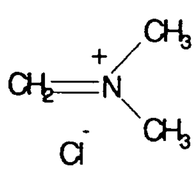

(XI)

gegebenenfalls am Rückfluß erhitzt, wobei das intermediär auftretende Zwischenprodukt der Formel (XII)

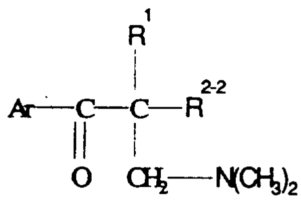

(XII)

gegebenenfalls isoliert und anschließend in einer zweiten Stufe in einem polaren organischen Lösungsmittel, vorzugsweise einem Alkohol bei Temperaturen von 20 bis 80 °C insbesondere bei 30 bis 60 °C mit Hydrazin-Hydrat zu Verbindungen der Formel (II) cyclisiert.

Verbindungen der Formel (IIa), in welcher $R^3$ für den Rest $-CH_2-N(CH_3)_2$ und $R^4$ für Wasserstoff steht, werden erhalten, indem man zunächst in einer 1. Stufe Verbindungen der Formel (VId)

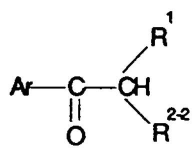

in welcher

Ar und $R^1$ die oben angegebene Bedeutung haben und

$R^{2-2}$ für Wasserstoff, Alkyl oder Alkoxycarbonyl steht,

in einem polaren organischen Lösungsmittel, vorzugsweise Acetonitril, bei Temperaturen von 10 bis 100 °C, insbesondere bei 20 bis 80 °C mit 2 Mol N,N-Dimethylmethylenimmoniumchlorid der Formel (XI)

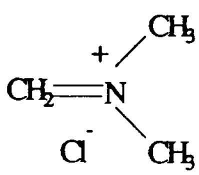

(XI)

gegebenenfalls am Rückfluß erhitzt, wobei das intermediär auftretende Zwischenprodukt der Formel (XIIa)

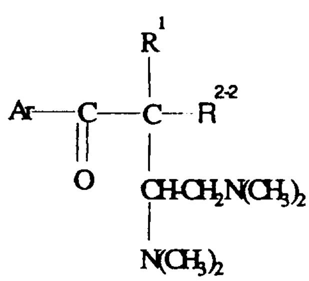

(XIIa)

gegebenenfalls isoliert und anschließend in einer zweiten Stufe in einem polaren organischen Lösungsmittel, vorzugsweise einem Alkohol bei Temperaturen von 20 bis 80 °C insbesondere bei 30 bis 60 °C mit Hydrazin-Hydrat zu Verbindungen der Formel (II) cyclisiert (vgl. auch das Herstellungsbeispiel).

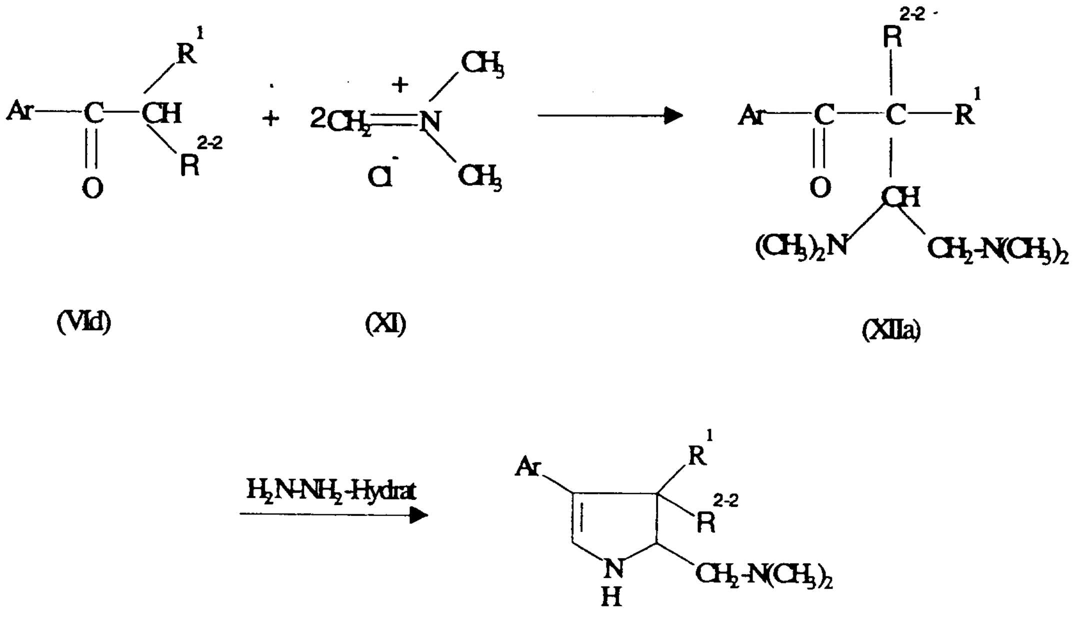


(VId) (XI) (XIIa)

(II-a)

Die Verbindungen der Formel (VIa) und (VId) sind teilweise neu. Sie werden durch Umsetzung der Verbindungen der Formel (X)

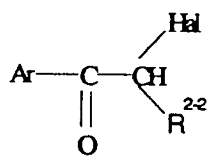

(X)

worin

- $R^{2-2}$ für Wasserstoff, Alkyl oder Alkoxycarbonyl steht,
- Ar die oben angegebene Bedeutung hat und
- Hal für Halogen, insbesondere Brom steht,

mit der Verbindung $R^1$-H unter Zuhilfenahme einer organischen oder anorganischen Base unter Halogenwasserstoffeliminierung hergestellt. Bei den Verbindungen der Formel (X) und $R^1$-H handelt es sich um bekannte Stoffe.

Bei der Durchführung des erfindungsgemäßen Verfahrens gemäß der Verfahrensvariante (B) werden Verbindungen der Formel (IV)

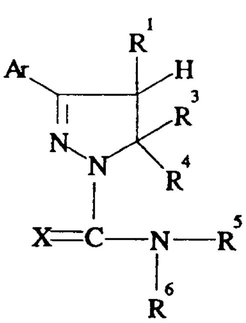

(IV)

in welcher X, Ar, $R^1$, $R^3$, $R^4$, $R^5$ und $R^6$ die oben angegebene Bedeutung besitzen mit einer starken Base, vorzugsweise einer metallorganischen Verbindung, insbesondere mit Butyl-Lithium im inerten organischen Lösungsmittel bei Temperaturen von -50 bis 0°C, insbesondere von -30°C bis -15°C, gegebenenfalls in Anwesenheit einer Schutzgasatmosphäre insbesondere Edelgasatmosphäre wie z.B. Argon umgesetzt und anschließend mit einem Halogenid Hal-$R^{2-1}$

worin

$R^{2-1}$ für Alkyl, gegebenenfalls substituiertes Cycloalkyl, Halogenalkyl, Alkoxycarbonyl oder Halogenalkylthio steht,

bei 0 bis 60°C, insbesondere bei -10 bis -40°C umgesetzt und durch Zusatz von Wasser und Extraktion mit Ether in üblicher Weise aufgearbeitet.

Man erhält dann Verbindungen der Formel (XIII)

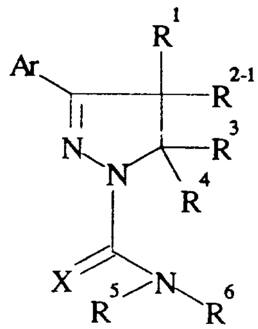

(XIII)

wobei

X, Ar, $R^1$, $R^{2-1}$, $R^3$, $R^4$, $R^5$ und $R^6$ die oben angegebenen Bedeutungen besitzen.

Die Wirkstoffe eignen sich zur Bekämpfung von tierischen Schädlingen, vorzugsweise Arthropoden und Nematoden, insbesondere Insekten und Spinnentieren, die in der Landwirtschaft, in der Tierhaltung, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa sppl. Locusta migratoria migratorioides, Melanoplus differentialis, Schistocerca gregaria. Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis,

Ephestia kuehniella, Galleria mellorella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrthynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Hydrotaea spp., Haematobia spp., Glossina spp., Melophagus spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp., Ctenocephalides spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Ornithonyssus spp., Eriophyes ribis, Phyllocoptruta oleivora, Boophilus spp., Rhipicephalus spp., Amblyomma spp., Hyalomma spp., Ixodes spp., Dermacentor spp., Haemaphysalis spp., Otobius spp., Psoroptes spp., Chorioptes spp., Sarcoptes spp., Psorergates spp., Demodex spp., Notoedres spp., Tarsonemus spp., Bryobia praetiosa, Panonychus spp., Tetranychus spp..

Die erfindungsgemäßen Wirkstoffe wirken nicht nur gegen Pflanzen-, Hygiene-und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, - Fliegen (stechend und leckend), parasitierende Fliegenlarven, Läuse, Haarlinge, Federtinge, Flöhe und endoparasitisch lebende Würmer.

Sie sind gegen normalsensible und resistente Arten und Stämme sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meeretttichblattkäfers (Phaedon cochleariae) oder gegen die Tabakknospenraupe (Heliothis virescens) einsetzen.

Darüberhinaus lassen sie sich auch mit besonders gutem Erfolg zur Bekämpfung von parasitisch lebenden Warmblüterschädlingen, wie beispielsweise gegen die Larven der Goldfliege (Lucilia cuprina), einsetzen.

Die Wirkstoffe können in die übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide,

Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiemittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halshand, Ohrmarke), sowie eine Anwendung in Form der sogenannten Umgebungsbehandlung möglich.

Die nachfolgenden Beispiele beschreiben die Herstellung und die Verwendung erfindungsgemäßer Wirkstoffe ohne sie darauf zu beschränken.

Herstellungsbeispiele

Beispiel 1:

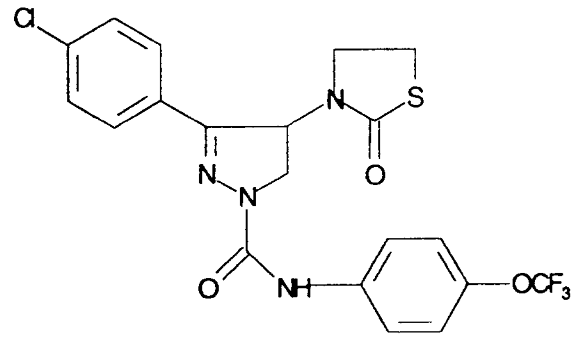

3,66 g (0,013 Mol) 3-(4'-Chlorphenyl)-4-(thiazolidin-2''-on-3''-yl)-4,5-dihydropyrazol werden in 20 ml Acetonitril (wasserfrei) bei 30-40°C klar gelöst. Anschließend werden 2,4 g (0,012 Mol) 4-Trifluormethoxy-phenylisocyanat und 2 Tropfen Triethylamin zugesetzt. Man läßt die Reaktionsmischung noch 1 Stunde bei Raumtemperatur stehen, zieht das Lösungsmittel im Vakuum ab und versetzt den Rückstand mit 20 ml Diethylether. Nach 1 bis 2 Stunden wird der entstandene Niederschlag abgesaugt.

Man erhält 0,8 g (14% der Theorie) 3-(4'-Chlorphenyl)-4-(thiazolidin-2''-on-3''-yl)-4,5-dihydro-1-pyrazolcarbonsäure-(4-trifluormethoxy-anilid) als farblose Kristalle mit dem Schmelzpunkt 219°C.

Beispiel 2

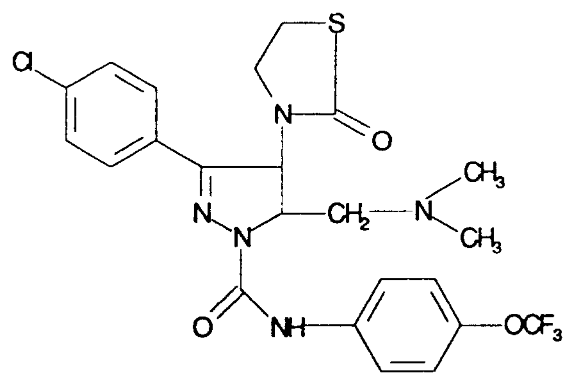

("Eintopfverfahren")
4,5 g (0,0176 Mol) $\alpha$-(Thiazolidin-2-on-3-yl)-4'-chloracetophenon werden in 100 ml Acetonitril (wasserfrei) gegebenenfalls unter Erwärmung klar gelöst und dann 4,5 g (0,048 Mol) N,N-Dimethylmethylenimmoniumchlorid zugesetzt. Die Reaktionsmischung wird 3 Stunden am Rückfluß erhitzt, bis laut DC-Kontrolle kein Ausgangsmaterial mehr erkennbar ist. Bei 40°C werden dann 10 ml Hydrazinhydrat zugesetzt und 1 Stunde bei Raumtemperatur nachgerührt. Die Reaktionsmischung wird im Vakuum eingeengt, der Rückstand mit 200 ml Wasser versetzt und mit 200 ml Diethylether extrahiert Die organische Phase wird abgetrennt, getrocknet und im Vakuum eingeengt.

Man erhält 3,6 g 3(4'-Chlorphenyl)-4-(thiazolidin-2''-on-3''-yl)-5-dimethylaminomethyl-4,5-dihydropyrazol als orangefarbiges Öl, das anschließend in 20 ml Acetonitril (wasserfrei) gelöst und mit 2,2 g 4-Trifluormethyl-phenylisocyanat und 1 Tropfen Triethylamin versetzt wird. Man laßt die Reaktionsmischung noch 1 Stunde bei Raumtemperatur stehen, zieht das Losungsmittel im Vakuum ab und versetzt den Rückstand mit 20 ml Diethylether. Nach 1 bis 2 Stunden wird der entstandene Niederschlag isoliert.

Man erhält 1,1 g 3-(4'-Chlorphenyl)-4-(thiazolidin-2''-on-3''-yl)-5-dimethylaminomethyl-4,5-dihydro-1-pyrazolcarbonsäure-(4-trifluormethylanilid) als farblose Kristalle mit dem Schmelzpunkt 172°C.

Analog zu den Beispielen 1 und 2 und gemaß den allgemeinen Angaben zur Herstellung werden die nachfolgend in Tabelle 2 aufgeführten Endprodukte der Formel (I) erhalten.

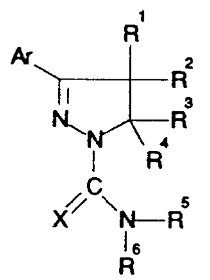

(I)

Tabelle 2

| Bsp. -Nr. | Ar | $R^1$ | $R^2$ | $R^3$ | $R^4$ | $R^5$ | $R^6$ | X | physikalische Konst. [°C] |
|---|---|---|---|---|---|---|---|---|---|
| 3 | 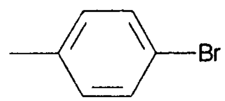 | 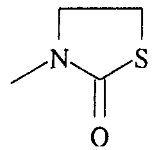 | H | H | H | H | 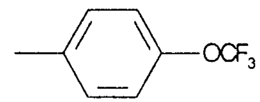 | O | Fp.: 228 |
| 4 | 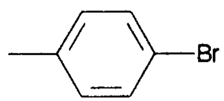 | 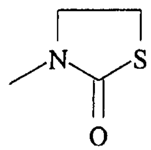 | H | H | H | H | 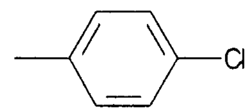 | O | Fp.: > 250 |
| 5 | 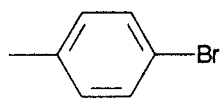 | 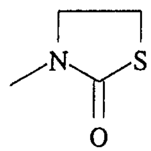 | H | H | H | H | 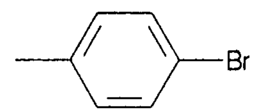 | O | Fp.: > 250 |
| 6 | 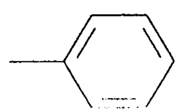 | 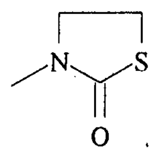 | H | H | H | H | 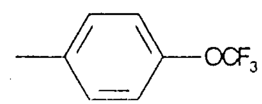 | O | Fp.: 221 |

Tabelle 2 - Fortsetzung

| Bsp. -Nr. | Ar | $R^1$ | $R^2$ | $R^3$ | $R^4$ | $R^5$ | $R^6$ | X | physikalische Konst. [°C] |
|---|---|---|---|---|---|---|---|---|---|
| 7 | 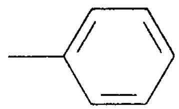 | 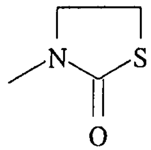 | H | H | H | H | 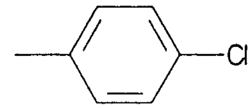 | O | Fp.: 234 |
| 8 | 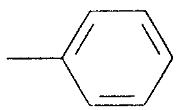 | 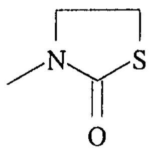 | H | H | H | H | 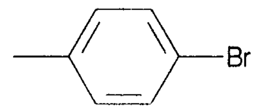 | O | Fp.: 249 |
| 9 | 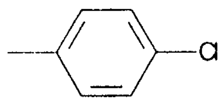 | 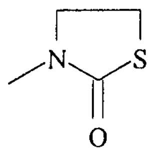 | H | $-CH_2N(CH_3)_2$ | H | H | 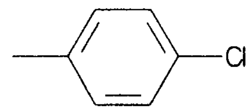 | O | Fp.: 206 |
| 10 | 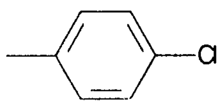 | 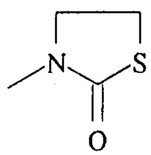 | H | $-CH_2N(CH_3)_2$ | H | H | 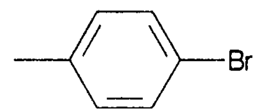 | O | Fp.: 217 |

Herstellung der Ausgangsverbindungen:

Beispiel (II-1)

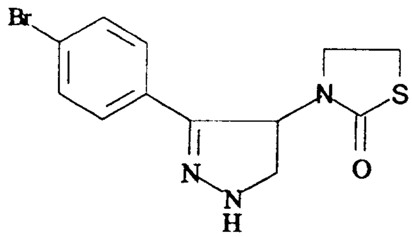

9,36 g (0,03 Mol) 4'-Brom-2-(thiazolidin-2-on-3-yl)-acrylophenon werden in 50 ml Ethanol klar gelöst. Bei 20 °C werden 3 ml Hydrazinhydrat zugegeben und anschließend noch 30 Minuten auf 30 °C erwärmt. Die Umsetzung wird dünnschichtchromatographisch kontrolliert). Anschließend wird der Ansatz sofort auf 200 ml Wasser gegeben und mit 200 ml Diethylether extrahiert. Die organische Phase wird abgetrennt über $MgSO_4$ getrocknet und das Lösungsmittel im Vakuum abgezogen.

Man erhält 7 g (72% der Theorie) 3-(4'-Bromphenyl)-4-(thiazolidin-2''-on-3''-yl)-4,5-dihydropyrazol als hellbraunen Feststoff ($^1$H-NMR*(DMSO)$\delta$ = 7,49-7,6(4H,m); 5,64-5,68(1H,m); 3,0-3,6(6H,m)).

Beispiel (II-2)

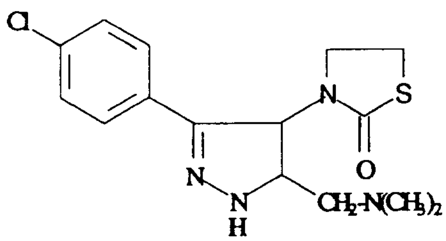

4,5 g (0,0176 Mol) $\alpha$-(Thiazolidin-2-on-3-yl)-4'-chloracetophenon werden in 100 ml Acetonitril (wasserfrei) gegebenenfalls unter Erwärmung klar gelöst und dann 4,5 g (0,048 Mol) N,N-Dimethylmethylenimmoniumchlorid zugesetzt. Die Reaktionsmischung wird 3 Stunden am Rückfluß erhitzt, bis laut DC-Kontrolle kein Ausgangsmaterial mehr erkennbar ist. Bei 40 °C werden dann 10 ml Hydrazinhydrat zugesetzt und 1 Stunde bei Raumtemperatur nachgerührt. Die Reaktionsmischung wird im Vakuum eingeengt, der Rückstand mit 200 ml Wasser versetzt und mit 200 ml Diethylether extrahiert. Die organische Phase wird abgetrennt, getrocknet und im Vakuum eingeengt.

Man erhält 3,6 g 3-(4'-Chlorphenyl-4-(thiazolidin-2''-on-3''-yl)-5-dimethylaminomethyl-4,5-dihydropyrazol als orangefarbenes Öl.

Analog zu den Beispielen (II-1) und (II-2) und gemäß den allgemeinen Angaben zur Herstellung werden die nachfolgend in Tabelle 3 aufgeführten Ausgangsverbindungen der Formel (II) erhalten:

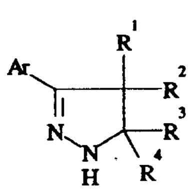 (II)

Tabelle 3

| Bsp.-Nr. | Ar | $R^1$ | $R^2$ | $R^3$ | $R^4$ | physikalische Konst. ($^1$H-NMR*δ) |
|---|---|---|---|---|---|---|
| II-3 | 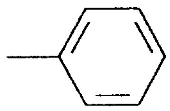 | 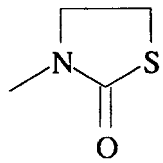 | H | H | H | 5,67-5,71 (1H, m); 3,0-3,6 (6H, m) |
| II-4 | 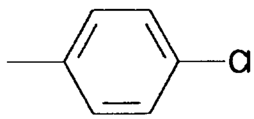 | 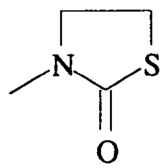 | H | H | H | 5,66-5,69 (1H, m); 3,0-3,6 (6H, m) |
| II-5 | 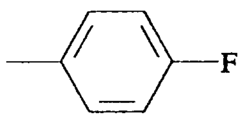 | 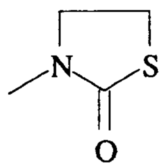 | H | H | H | Öl |
| II-6 | 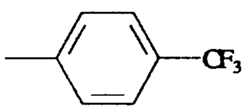 | 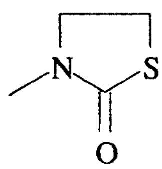 | H | H | H | Öl |

Tabelle 3 - Fortsetzung

| Bsp.-Nr. | Ar | $R^1$ | $R^2$ | $R^3$ | $R^4$ | physikalische Konst. ($^1$H-NMR*δ) |
|---|---|---|---|---|---|---|
| II-7 | 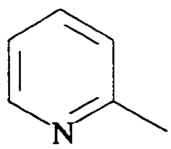 | 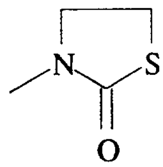 | H | H | H | Öl |
| II-8 | 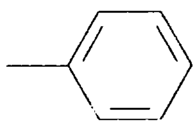 | 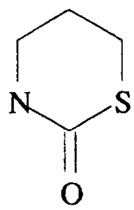 | H | H | H | Öl |
| II-9 | 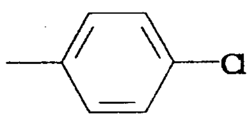 | 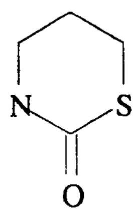 | H | H | H | Öl |
| II-10 | 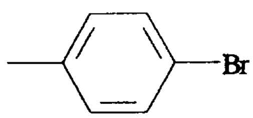 | 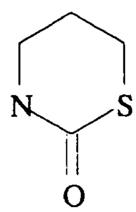 | H | H | H | Öl |

* Die $^1$H-NMR Spektren wurden in deuteriertem Dimethylsulfoxid $(CD_3)_2SO$ mit Tetramethylsilan (TMS) als innerem Standard aufgenommen. Angegeben ist die chemische Verschiebung δ in ppm.

Herstellung der Vorprodukte:

Beispiel (VIb-1)

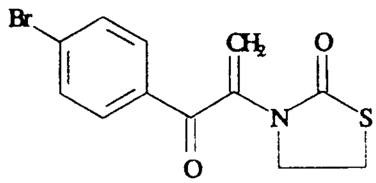

12 g (0,04 Mol) α-(Thiazolidin-2-on-3-yl)-4-bromacetophenon werden in 100 ml Methanol gelöst, 40 ml 37%ige wäßrige Formalinlösung, 1 ml Piperidin und 1 ml Eisessig zugegeben und dann 3 Stunden auf 60 °C erwärmt. Anschließend wird das Lösungsmittel im Vakuum eingeengt, mit 200 ml Wasser versetzt und mit 300 ml Diethylether extrahiert. Die organische Phase wird abgetrennt, getrocknet und das Lösungsmittel im Vakuum abgezogen.

Man erhält 9,5 g (76% der Theorie) 4'-Brom-2(thiazolidin-2-on-3-yl)-acrylophenon als rotbraunes Öl, das ohne weitere Reinigung weiter verwendet wird.

($^1$H-NMR*(DMSO):δ = 7,7(4H,s); 5,32(1H,d); 5,13 (1H,d); 4,05 (2H,t); 3,53 (2H, t)).

Analog zu Beispiel (VIb-1) und gemäß den allgemeinen Angaben zur Herstellung werden die nachfolgend in Tabelle 4 aufgeführten Vorprodukte der Formel (VIb) erhalten:

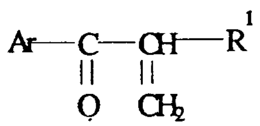 (VIb)

* Die $^1$H-NMR Spektren wurden in deuteriertem Dimethylsulfoxid $(CD_3)_2SO$ mit Tetramethylsilan (TMS) als innerem Standard aufgenommen. Angegeben ist die chemische Verschiebung δ in ppm.

Tabelle 4

| Bsp.-Nr. | Ar | $R^1$ | physikalische Konstanten |
|---|---|---|---|
| (VIb-2) | 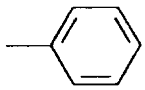 | 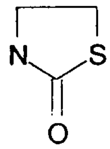 | Öl |
| (VIb-3) | 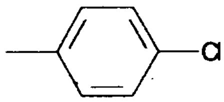 | 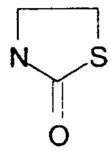 | Öl |
| (VIb-4) | 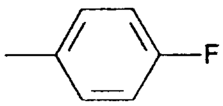 | 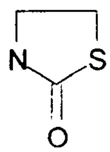 | Öl |
| (VIb-5) | 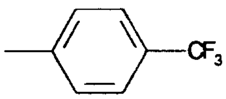 | 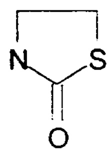 | Öl |
| (VIb-6) | 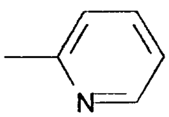 | 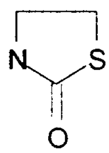 | Öl |

Tabelle 4 - Fortsetzung

| Bsp.-Nr. | Ar | $R^1$ | physikalische Konstanten |
|---|---|---|---|
| (VIb-7) | 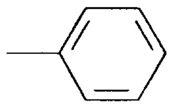 | 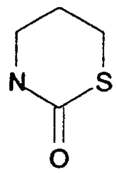 | Öl |
| (VIb-8) | 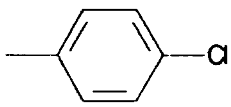 | 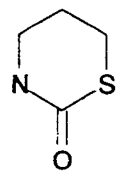 | Öl |
| (VIb-9) | 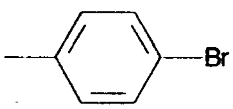 | 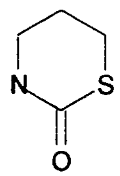 | Öl |

Beispiel (VIa-1)

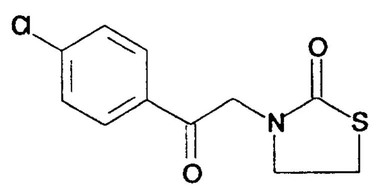

1,5 g (0,065 Mol) Natriumhydrid werden in 20 ml DMF (wasserfrei) suspendiert und dann 5,15 g (0,05 Mol) Thiazolidin-2-on gelöst in 50 ml DMF (wasseifrei) bei 20-50 °C zugetropft und dann noch 30 Minuten bei Raumtemperatur nachgerührt. Anschließend werden 11,68 g (0,05 Mol) 4-Chlorphenacylbromid gelöst in 20 ml DMF wasserfrei zugetropft und noch 30 Minuten bei Raumtemperatur nachgerührt. Der Reaktionsansatz wird im Vakuum auf die Hälfte eingeengt, mit 300 ml Wasser versetzt und mit Essigester extrahiert. Die organische Phase wird abgetrennt über $MgSO_4$ getrocknet und anschließend das Lösungsmittel im Vakuum abgezogen. Der Rückstand wird mit etwas Diethylether kristallin gerieben.

Man erhält 20 g (76,5% der Theorie) α-(Thiazolidin-2-on-3-yl)-4-chloracetophenon als hellgelben Feststoff.

($H^2$ NMR*(DMSO): $\delta$ = 7,55-8,0 (4H,m), 4,85 (2H,s), 3,33-3,74 (4H,m)) Analog zu Beispiel (VIa-1) und gemäß den allgemeinen Angaben zur Herstellung werden die nachfolgend in Tabelle 5 aufgeführten Vorprodukte der Formel (VIa) erhalten:

* Die $^1H$-NMR Spektren wurden in deuteriertem Dimethylsulfoxid $(CD_3)_2SO$ mit Tetramethylsilan (TMS) als innerem Standard aufgenommen. Angegeben ist die chemische Verschiebung $\delta$ in ppm.

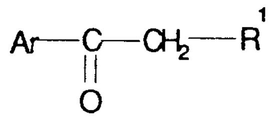

<u>Tabelle 5</u>

| Bsp.-Nr. | Ar | $R^1$ | physikalische Konstanten |
|---|---|---|---|
| (VIa-2) | 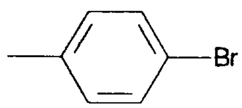 | 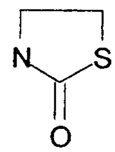 | Öl |
| (VIa-3) | 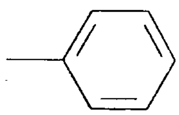 | 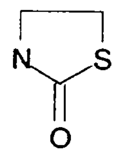 | Öl |
| (VIa-4) | 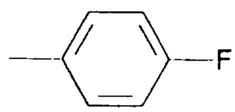 | 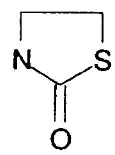 | |
| (VIa-5) | 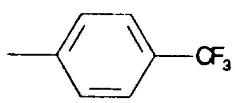 | 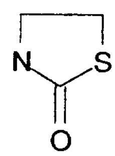 | |
| (VIa-6) | 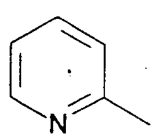 | 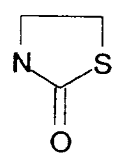 | |

Tabelle 5 - Fortsetzung

| Bsp.-Nr. | Ar | $R^1$ | physikalische Konstanten |
|---|---|---|---|
| (VIa-7) | 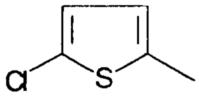 | 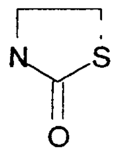 | |
| (VIa-8) | 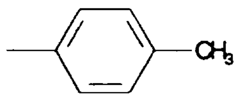 | 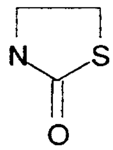 | |
| (VIa-9) | 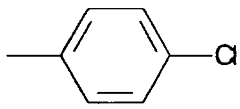 | 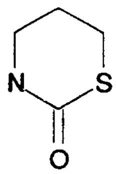 | |
| (VIa-10) | 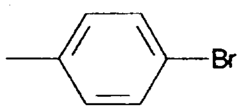 | 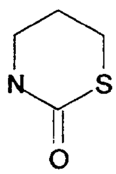 | |
| (VIa-11) | 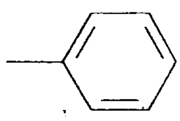 | 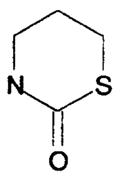 | |

## Anwendungsbeispiele

In den folgenden Anwendungsbeispielen wurden die nachstehend aufgeführten Verbindungen als Vergleichssubstanzen eingesetzt.

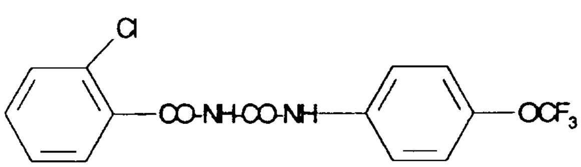

(A)

Triflumuron = 2-Chlor-N[[[4-(trifluormethoxy)-phenyl]-amino]-carbonyl]-benzamid (bekannt aus: DE-A 2601780)

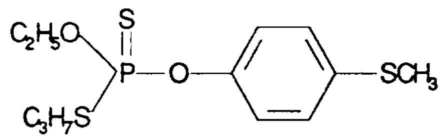 (B)

Sulprofos = O-(Ethyl-O-(4-methylthio)-phenyl)-S-propyldithiophosphat (bekannt aus: DE-A 2111414)

Beispiel A

Phaedon-Larven-Test

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 2, 3, 6 und 10.

Beispiel B

Heliothis virescens-Test

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) werden durch Tauchen in die Wirkstoffzubereitung der gewünschten Konzentration behandelt und mit der Tabakknospenraupe (Heliothis virescens) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesen Test zeigen z.B. die folgenden Verbindungen der Herstellungsbeispiele überlegene Wirksamkeit gegenüber dem Stand der Technik: 2 und 3.

Beispiel C

Blowfly-Larven-Test

Testtiere: Lucilia cuprina-Larven
Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Emulsionskonzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird die Wirksamkeit der Wirkstoffzubereitung ermittelt. Dabei bedeutet 100%, daß alle Blowfly-Larven abgetötet wurden; 0% bedeutet, daß keine Blowfly-Larven abgetötet wurden.

Bei diesem Test zeigt beispielsweise die Verbindung 3 der Herstellungsbeispiele eine hervorragende Wirkung bei 100 ppm.

**Patentansprüche**

**1.** Substituierte 4,5-Dihydro-1-pyrazolcarbonsäureanilide der allgemeinen Formel (I),

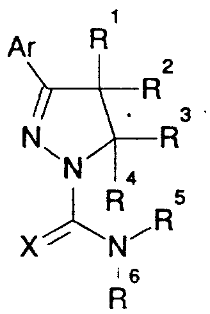

(I)

in welcher

| | |
|---|---|
| $R^1$ | für einen gegebenenfalls substituierten Heterocyclus aus der Reihe |

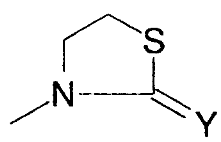

oder

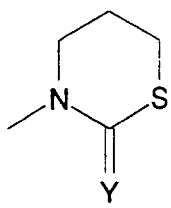

| | |
|---|---|
| | steht<br>in welcher |
| Y | für Sauerstoff oder Schwefel steht, |
| $R^2$ | für Wasserstoff, Alkyl, gegebenenfalls substituiertes Cycloalkyl, Halogenakyl, Halogenalkylthio oder Alkoxycarbonyl steht, |
| $R^3$ | für Wasserstoff, Alkyl oder für eine Gruppierung |

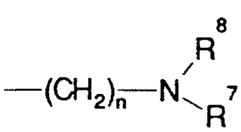

| | |
|---|---|
| | oder $-(CH_2)_n-O-R^9$ steht<br>worin |
| $R^7$ und $R^8$ | jeweils unabhängig voneinander für Wasserstoff, Alkyl oder Aryl stehen und |
| $R^9$ | für Wasserstoff, Alkyl oder Aryl steht und |
| n | für die Zahlen 1 bis 6 steht, |
| $R^4$ | für Wasserstoff oder Alkyl steht, |
| $R^5$ | für Wasserstoff, Alkyl, Phenyl oder Alkylthio steht, |
| $R^6$ | für gegebenenfalls substituiertes Alkyl, gegebenenfalls substituiertes Cycloalkyl oder für den Rest |

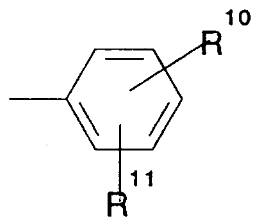

steht,

worin $R^{10}$ und $R^{11}$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, gegebenenfalls substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl stehen oder wobei $R^{10}$ und $R^{11}$ zusammen für einen bivalenten gegebenenfalls ein oder zwei Sauerstoffatome enthaltenden und gegebenenfalls substituierten Rest stehen,

X für Sauerstoff oder Schwefel steht und

Ar für gegebenenfalls substituiertes Aryl oder für einen gegebenenfalls substituierten und/oder gegebenenfalls anellierten Heterocyclus steht.

2. Verfahren zur Herstellung der substituierten 4,5-Dihydro-1-pyrazolcarbonsäureanilide der allgemeinern Formel (I)

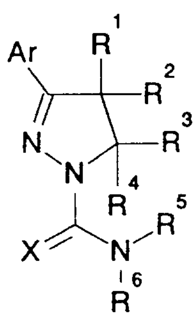

(I)

in welcher

$R^1$ für einen gegebenenfalls substituierten Heterocyclus aus der Reihe

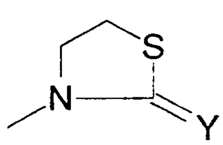

oder

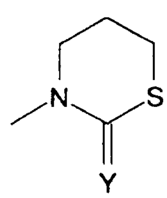

steht

in welcher

Y für Sauerstoff oder Schwefel steht,

$R^2$ für Wasserstoff, Alkyl, gegebenenfalls substituiertes Cycloalkyl, Halogenalkyl, Halogenalkylthio oder Alkoxycarbonyl steht,

$R^3$ für Wasserstoff, Alkyl oder für eine Gruppierung

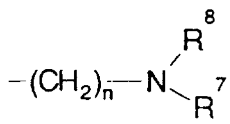

oder $-(CH_2)_n-O-R^9$ steht
worin

- $R^7$ und $R^8$ jeweils unabhängig voneinander für Wasserstoff, Alkyl oder Aryl stehen und
- $R^9$ für Wasserstoff, Alkyl oder Aryl steht und
- n für die Zahlen 1 bis 6 steht,
- $R^4$ für Wasserstoff oder Alkyl steht,
- $R^5$ für Wasserstoff, Alkyl, Phenyl oder Alkylthio steht,
- $R^6$ für gegebenenfalls substituiertes Alkyl, gegebenenfalls substituiertes Cycloalkyl oder für den Rest

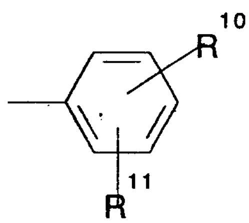

steht,

worin $R^{10}$ und $R^{11}$ gleich oder verschieden sein können und für Wasserstoff, Halogen, Alkyl, Nitro, Cyano, Halogenalkyl, Alkoxy, Halogenalkoxy, Alkylthio, Halogenalkylthio, gegebenenfalls substituiertes Phenoxy, gegebenenfalls substituiertes Mono- oder Dialkylamino, gegebenenfalls substituiertes Cycloalkyl, Alkoxycarbonyl, gegebenenfalls substituiertes Arylthio, Alkenyloxy, Alkinyl, Alkylthionyl, Alkylsulfonyl, Halogenalkylthionyl, Halogenalkylsulfonyl, Halogenalkoxycarbonyl stehen oder wobei $R^{10}$ und $R^{11}$ zusammen für einen bivalenten gegebenenfalls ein oder zwei Sauerstoffatome enthaltenden und gegebenenfalls substituierten Rest stehen,

- X für Sauerstoff oder Schwefel steht und
- Ar für gegebenenfalls substituiertes Aryl oder für einen gegebenenfalls substituierten und/oder gegebenenfalls anellierten Heterocyclus steht,

dadurch gekennzeichnet, daß man

(A) zum Erhalt von 4,5-Dihydro-1-pyrazolcarbonsäureaniliden der Formel (I), in welcher $R^5$ für Wasserstoff steht, Pyrazolinderivate der Formel (II)

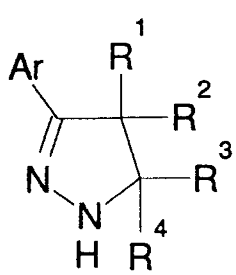

(II)

in welcher

$R^1$, $R^2$, $R^3$, $R^4$ und Ar die oben angegebene Bedeutung besitzen,

mit Isocyanaten bzw. Isothiocyanaten der Formel (III)

$X=C=N-R^6$ (III)

in welcher

X und $R^6$ die oben angegebene Bedeutung haben,

gegebenenfalls in Gegenwart von Basen umsetzt oder wenn man

(B) zum Erhalt von Pyrazolinderivaten der Formel (I), in welcher $R^2$ für Alkyl, Cycloalkyl, Halogenalkyl, Halogenalkylthio oder Alkoxycarbonyl steht, Verbindungen der Formel (IV)

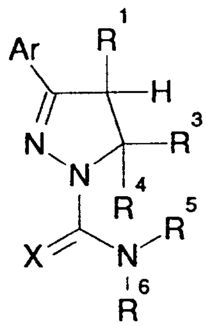

(IV)

mit Verbindungen der Formel (V)

Hal-$R^{2-1}$ (V)

in welcher

Hal für Halogen steht und

$R^{2-1}$ für Alkyl, gegebenenfalls substituiertes Cycloalkyl, Halogenalkyl, Halo-genalkylthio oder Alkoxycarbonyl steht,

in wasserfreiem Medium unter Zusatz einer starken Base umsetzt.

**3.** Substituierte 4,5-Dihydro-1-pyrazolcarbonsäureanilide der Formel (I) gemäß Anspruch 1, bei welchen

$R^1$ für einen unsubstituierten oder einfach bis mehrfach, gleich oder verschieden substituierten Heterocyclus aus der Reihe

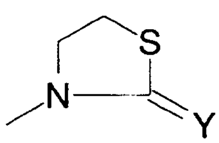

oder

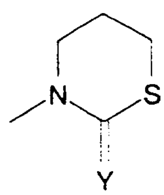

steht

in welcher

Y für Sauerstoff oder Schwefel steht,

und wobei folgende Substituenten infrage kommen: Alkyl ($C_1$-$C_6$), Alkoxy ($C_1$-$C_6$), Alkyl($C_1$-$C_6$)thio, Amino, Alkyl($C_1$-$C_6$)amino, Dialkyl($C_1$-$C_6$)amino und gegebenenfalls durch Halogen, Alkyl($C_1$-$C_4$), Alkoxy($C_1$-$C_4$), Halogenalkyl($C_1$-$C_4$), Halogenalkoxy($C_1$-$C_4$)- oder Halogenalkyl($C_1$-$C_4$)thio substituiertes Phenyl,

$R^2$ für Wasserstoff, Alkyl($C_1$-$C_6$), gegebenenfalls durch Halogen, Halogenalkyl($C_1$-$C_4$) substituiertes Cycloalkyl($C_3$-$C_7$); Halogenalkyl($C_1$-$C_4$)thio oder Alkoxy($C_1$-$C_6$)-carbonyl steht,

$R^3$ für Wasserstoff, Alkyl($C_1$-$C_6$) oder für eine Gruppierung

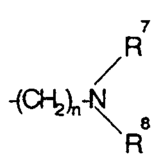

oder $-(CH_2)_n-OR^9$ steht,
worin

$R^7$ und $R^8$ jeweils unabhängig voneinander für Wasserstoff, Alkyl($C_1$-$C_6$) oder für gegebenenfalls durch Alkyl($C_1$-$C_4$), Alkoxy($C_1$-$C_4$), Alkyl($C_1$-$C_4$)thio, Halogen, Halogenalkyl($C_1$-$C_4$), Halogenalkoxy($C_1$-$C_4$) oder Halogenalkyl($C_1$-$C_4$)thio substituiertes Phenyl stehen und

$R^9$ für Wasserstoff, Alkyl($C_1$-$C_6$) oder gegebenenfalls substituiertes Phenyl steht, wobei als Substituenten die für $R^8$ aufgeführten Phenylsubstituenten infrage kommen und

n für die Zahlen 1 bis 4 steht,

$R^4$ für Wasserstoff oder Alkyl($C_1$-$C_6$) steht,

$R^5$ für Wasserstoff, Alkyl($C_1$-$C_6$), Phenyl oder Alkyl($C_1$-$C_4$)thio steht,

$R^6$ für gegebenenfalls durch Halogen, Halogenalkyl($C_1$-$C_4$), Halogenalkoxy($C_1$-$C_4$) substituiertes Alkyl($C_1$-$C_6$), für gegebenenfalls durch Halogen, Halogenalkyl($C_1$-$C_4$), Halogenalkoxy($C_1$-$C_4$) substituiertes Cycloalkyl($C_3$-$C_7$) oder für den Rest

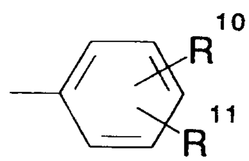

steht,
wobei

$R^{10}$ und $R^{11}$ gleich oder verschieden sein können und für Halogen, Alkyl($C_1$-$C_6$), Nitro, Cyano, Halogenalkyl($C_1$-$C_4$), Alkoxy($C_1$-$C_6$), Halogenalkoxy($C_1$-$C_4$), Alkyl($C_1$-$C_4$)thio, Halogenalkyl($C_1$-$C_4$)thio, gegebenenfalls durch Halogen, Halogenalkyl($C_1$-$C_4$), Alkoxy($C_1$-$C_4$), Alkyl($C_1$-$C_4$) substituiertes Phenoxy oder Phenylthio, gegebenenfalls durch Halogen, Alkoxy($C_1$-$C_4$), Halogenalkyl($C_1$-$C_4$) substituiertes Mono- oder Dialkylamino mit jeweils 1 bis 6 Kohlenstoffatomen im Alkylrest, gegebenenfalls durch Alkyl($C_1$-$C_4$), Alkoxy($C_1$-$C_4$), Halogen, Alkyl($C_1$-$C_4$)thio substituiertes Cycloalkyl($C_3$-$C_7$), Alkoxy($C_1$-$C_4$)carbonyl, Alkenyl($C_2$-$C_6$)oxy, Alkinyl($C_2$-$C_6$), Alkyl($C_1$-$C_4$)thionyl, Alkyl($C_1$-$C_4$)sul-fonyl, Halogenalkyl($C_1$-$C_4$)thionyl, Halogenalkyl($C_1$-$C_4$)sulfonyl, Halogenalkoxy($C_1$-$C_4$)-carbonyl stehen oder wobei

$R^{10}$ und $R^{11}$ zsammen für einen der folgenden bivalenten Reste stehen

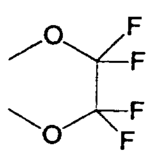 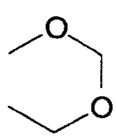 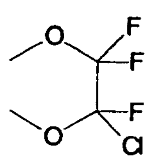 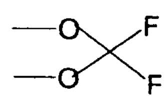

X für Sauerstoff oder Schwefel steht und

Ar für gegebenenfalls substituiertes Phenyl oder Naphthyl steht , wobei als Substituenten genannt seien: Alkyl, Halogen, Halogenalkyl, Alkoxy, Alkylthio, Halogenalkoxy, Halogenalkylthio, Alkoxycarbonyl, Halogenalkoxycarbonyl, gegebenenfalls substituiertes Aryloxy, gegebenenfalls substituiertes Arylthio, Alkenyloxy, Alkinyl, Alkylthionyl, Alkylsulfonyl, Halogenalkylthionyl, Halogenalkylsulfonyl, Nitro, Cyano oder wobei zwei benachbarte Positionen durch einen durch Halogen substituierten 3,4-Methylendioxyl- oder 3,4-Ethylendioxylrest miteinander verbunden sind;
weiterhin steht Ar für einen unsubstituierten oder einfach bis mehrfach, gleich oder verschieden substituierten, gegebenenfalls benzoanellierten heteroaromatischen 5- oder 6-gliedrigen Ring, der gleiche oder verschiedene, ein oder mehrere Heteroatome wie Sauerstoff, Schwefel und Stickstoff enthält, wobei als Substituenten die oben für Phenyl und Naphthyl aufgeführten Substituenten infrage kommen.

4. Schädlingsbekämpfüngsmittel, gekennzeichnet durch einen Gehalt an mindestens einem substituierten 4,5-Dihydro-1-pyrazolcarbonsäureanilid der Formel (I) gemäß Anspruch 1.

5. Verwendung von substituiertem 4,5-Dihydro-1-pyrazolcarbonsäureanilid der Formel (I) gemäß Anspruch 1 zur Bekämpfung von Schädlingen.

6. Verfahren zur Bekämpfung von Schädlingen, dadurch gekennzeichnet, daß man substituierte 4,5-Dihydro-1-pyrazolcarbonsäureanilide der Formel (I) gemäß Anspruch 1 auf Schädlinge und/oder ihren Lebensraum einwirken läßt.

7. Verfahren zur Herstellung von Schädlingsbekämpfüngsmitteln, dadurch gekennzeichnet, daß man substituierte 4,5-Dihydro-1-pyrazolcarbonsäureanilideder Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung
EP 93 11 5419

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,X | EP-A-0 466 408 (ROHM AND HAAS COMPANY) 15. Januar 1992<br>* Ansprüche 1,22 *<br>----- | 1,4 | C07D417/04<br>A01N47/38 |
| | | | **RECHERCHIERTE SACHGEBIETE (Int.Cl.5)** |
| | | | C07D<br>A01N |

Der vorliegende Recherchenbericht wurde für alle Patentansprüche erstellt

| Recherchenort | Abschlußdatum der Recherche | Prüfer |
|---|---|---|
| DEN HAAG | 14. Januar 1994 | VOYIAZOGLOU, D |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument